# EUROPEAN PATENT APPLICATION

(11) **EP 4 830 730 A2**
(43) Date of publication of application: **28.10.2026**
(21) Application number: 26204772.3
(22) Date of filing: 20.09.2024
(51) Int. Cl.: A61K 47/44

(54) **CAPSULES WITH SELF-EMULSIFYING DRUG DELIVERY SYSTEMS**

(62) Document number(s) of the earlier application(s) in accordance with Art. 76 EPC: 24787058.7 / 4 791 360
(71) Applicant: Fertin Pharma A/S, 7100 Vejle (DK)
(72) Inventor: Jensen, Sanne, 7100 Vejle (DK); Nikolajsen, Gitte Nykjaer, 7100 Vejle (DK); Wahlqvist, Benjamin Bugge, 7100 Vejle (DK)
(74) Representative: Kanved, Nicolai

(57) **Abstract**

The present invention relates to a capsule for peroral delivery of cannabinoids comprising a cannabinoid formulation contained in a hard shell or soft shell of the capsule. The formulates comprises: one or more self-emulsifying drug delivery systems (SEDDS) comprising one or more surfactants having a chemical structure that includes a polyethylene glycol (PEG) moiety in an amount of at least 20% by weight of the one or more SEDDS; and one or more cannabinoids comprising one or more isolated or synthetic cannabinoids in an amount of at least 10% by weight of the formulation.

## Description

### FIELD OF THE INVENTION

The invention relates to the field of cannabinoids. In particular, the invention relates to capsules with self-emulsifying drug delivery systems (SEDDS) for peroral delivery of cannabinoids, such as SEDDS including a relatively high amount of surfactants with a polyethylene glycol (PEG) moiety.

### BACKGROUND OF THE INVENTION

Various considerations are to be taken into account when formulating delivery vehicles for administration of cannabinoids. Specifically, certain systems for gastrointestinal delivery of cannabinoids would be expected to be degraded to a high degree and therefore resulting in less uptake in the gastrointestinal tract, such as the colon. Other systems for gastrointestinal delivery of cannabinoids would be expected to result in less cell uptake due to steric hindrance and thereby less uptake in the gastrointestinal tract, such as the small intestines.

Gastrointestinal delivery of cannabinoids may provide some advantages compared to oral mucosal uptake of cannabinoids, particularly in relation to uptake of cannabinoids in the small intestines or the colon of the gastrointestinal tract involving uptake on a short time scale, but also on a long time scale. However, certain challenges arise if the cannabinoids are to be administered in the oral cavity but are to be delivered to mucosal surfaces in the gastrointestinal tract in order to benefit from improved uptake. Considerations such as the capability to obtain a suitable uptake in the gastrointestinal tract apply.

Although oral administration of cannabinoids is a common route of administration, less attention has been given in the prior art to systems that may allow a high amount of cannabinoids to be administered to patients in need thereof. Particularly, less attention has been given to systems for cannabinoids that allow a high load of cannabinoids to be delivered in the gastrointestinal tract, which at the same time offers an improved uptake of cannabinoids in the gastrointestinal tract.

Accordingly, there is a need in the prior art for systems that may accommodate increased uptake of cannabinoids in the gastrointestinal tract, such as the small intestines or the colon. At the same time, there is a need for systems that provide suitable stability of cannabinoids, when for instance uptake of cannabinoids is to occur in the small intestines or colon.

### SUMMARY OF THE INVENTION

Accordingly, there is provided a capsule for peroral delivery of cannabinoids comprising a cannabinoid formulation contained in a hard shell or soft shell of the capsule, the formulation comprising: one or more self-emulsifying drug delivery systems (SEDDS) comprising one or more surfactants having a chemical structure that includes a polyethylene glycol (PEG) moiety in an amount of at least 20% by weight of the one or more SEDDS; and one or more cannabinoids comprising one or more isolated or synthetic cannabinoids in an amount of at least 10% by weight of the formulation.

In accordance with the invention, it was not expected that a high load of cannabinoids could be obtained and at the same time that the formulation could serve to deliver the cannabinoids to the gastrointestinal target without the challenges disclosed in the prior art. The challenges are particularly the case if a high load of cannabinoids are to be present and accordingly a high load of cannabinoids in SEDDS is present in the formulation.

Specifically, gastrointestinal delivery of cannabinoids would be expected to result in less cell uptake due to steric hindrance of PEG contained in the SEDDS according to the invention and thereby less uptake in the gastrointestinal tract, such as the small intestines. The results obtained in rat studies revealed that an increase in both short term and long term uptake were in fact possible with the SEDDS containing a relatively high amount of surfactants having a chemical structure that includes a polyethylene glycol moiety (PEG) according to the invention, contrary to every expectation. This was highly surprising and considered a huge step towards establishing effective systems for delivery of cannabinoids to the gastrointestinal tract. Further comparison with Epidiolex^{®} (which is considered the current standard to compare with) revealed that the uptake was also significantly higher with the SEDDS according to the invention.

Additionally, the results revealed a significant increase in the Cmax (maximum concentration reached in rat studies) compared to SEDDS without containing a relatively high amount of surfactants having a chemical structure that includes a polyethylene glycol moiety (PEG) according to the invention. This was also very surprising and unexpected. Further comparison with Epidiolex^{®} (which is considered the current standard to compare with) revealed that also the Cmax was significantly higher with the SEDDS according to the invention.

Furthermore, the absolute bioavailability of the one or more cannabinoids according to the invention was seen from rat studies to be significantly higher compared to SEDDS that does not include a relatively high amount of polyethylene glycol moiety (PEG). Comparison with Epidiolex^{®} (which is considered the current standard to compare with) revealed that also the bioavailability was significantly higher with the SEDDS according to the invention. In the present context, "bioavailability" or "absolute bioavailability" is measured as the dose corrected blood plasma level relative to intravenous administration, i.e., intravenous administration would give 100% bioavailability.

Combined these benefits is considered to be a huge step towards establishing effective systems for delivery of cannabinoids to the gastrointestinal tract.

In some embodiments of the invention, the one or more isolated or synthetic cannabinoids is present in an amount of at least 15% by weight of the formulation.

In some embodiments of the invention, the one or more isolated or synthetic cannabinoids is present in a relative high amount by weight of the one or more SEDDS. This relative high amount was seen to be preferred in order to balance a high uptake in terms of plasma concentration and the necessity of the self-emulsifying systems to work in the best way. Additionally, a relative high presence of cannabinoids, such as CBD, in the SEDDS may be important in order to limit the content of SEDDS in the oral formulation.

In some embodiments of the invention, the one or more isolated or synthetic cannabinoids is present in an amount of at least 20% by weight of the formulation.

In some embodiments of the invention, the one or more isolated or synthetic cannabinoids is present in an amount of at least 22% by weight of the formulation.

In some embodiments of the invention, the one or more isolated or synthetic cannabinoids is present in an amount of at least 25% by weight of the formulation.

In some embodiments of the invention, the one or more isolated or synthetic cannabinoids is present in an amount of at least 27% by weight of the formulation.

In some embodiments of the invention, the one or more isolated or synthetic cannabinoids is present in an amount of at least 30% by weight of the formulation.

In some embodiments of the invention, the one or more isolated or synthetic cannabinoids is present in an amount of 20 to 50% by weight of the formulation.

In some embodiments of the invention, the one or more isolated or synthetic cannabinoids is present in an amount of 20 to 40% by weight of the formulation.

In some embodiments of the invention, the one or more isolated or synthetic cannabinoids is present in an amount of 20 to 35% by weight of the formulation.

In some embodiments of the invention, the one or more isolated or synthetic cannabinoids is present in an amount of 25 to 35% by weight of the formulation.

In some embodiments of the invention, the one or more isolated or synthetic cannabinoids is present in an amount of 25 to 40% by weight of the formulation.

In some embodiments of the invention, the one or more isolated or synthetic cannabinoids is present in an amount of 25 to 50% by weight of the formulation.

In some embodiments of the invention, the one or more isolated or synthetic cannabinoids is present in an amount of 10 to 50% by weight of the formulation.

In some embodiments of the invention, the one or more isolated or synthetic cannabinoids is present in an amount of 15 to 50% by weight of the formulation.

In some embodiments of the invention, the one or more isolated or synthetic cannabinoids is present in an amount of 10 to 35% by weight of the formulation.

In some embodiments of the invention, the one or more isolated or synthetic cannabinoids is present in an amount of 15 to 35% by weight of the formulation.

In some embodiments of the invention, the one or more SEDDS are present in an amount of at least 40% by weight of the formulation.

In some embodiments of the invention, the one or more SEDDS are present in an amount of at least 50% by weight of the formulation.

In some embodiments of the invention, the one or more SEDDS are present in an amount of at least 55% by weight of the formulation.

In some embodiments of the invention, the one or more SEDDS are present in an amount of at least 60% by weight of the formulation.

In some embodiments of the invention, the one or more SEDDS are present in an amount of 40 to 70% by weight of the formulation.

In some embodiments of the invention, the one or more SEDDS are present in an amount of 40 to 80% by weight of the formulation.

In some embodiments of the invention, the one or more SEDDS are present in an amount of 50 to 70% by weight of the formulation.

In some embodiments of the invention, the one or more SEDDS are present in an amount of 50 to 75% by weight of the formulation.

In some embodiments of the invention, the formulation in addition to any water-soluble agents present in the one or more SEDDS comprises less than 20% by weight of water-soluble agents by weight of the formulation.

In some embodiments of the invention, the formulation in addition to any water-soluble agents present in the one or more SEDDS comprises less than 15% by weight of water-soluble agents by weight of the formulation.

In some embodiments of the invention, the formulation in addition to any water-soluble agents present in the one or more SEDDS comprises less than 10% by weight of water-soluble agents by weight of the formulation.

In some embodiments of the invention, the formulation in addition to any water-soluble agents present in the one or more SEDDS does not comprise water-soluble agents.

In some embodiments of the invention, the formulation does not comprise sugar alcohol or sugars.

In some embodiments of the invention, the formulation does not comprise flavor. In some other embodiments of the invention, the formulation comprises flavor, such as in an amount of 1-10% by weight of the formulation.

In some embodiments of the invention, the one or more SEDDS and the one or more cannabinoids constitute at least 50% by weight of the formulation.

In some embodiments of the invention, the one or more SEDDS and the one or more cannabinoids constitute at least 60% by weight of the formulation.

In some embodiments of the invention, the one or more SEDDS and the one or more cannabinoids constitute at least 70% by weight of the formulation.

In some embodiments of the invention, the one or more SEDDS and the one or more cannabinoids constitute at least 80% by weight of the formulation.

In some embodiments of the invention, the one or more SEDDS and the one or more cannabinoids constitute at least 90% by weight of the formulation.

In some embodiments of the invention, the one or more SEDDS and the one or more cannabinoids constitute at least 95% by weight of the formulation.

In some embodiments of the invention, the one or more SEDDS and the one or more cannabinoids constitute 100% by weight of the formulation.

In some embodiments of the invention, the capsule comprises a hard shell. In some embodiments of the invention, the capsule comprises a soft shell. In some embodiments of the invention, the capsule is a two-piece hard gelatine capsule. In some embodiments of the invention, the capsule is a two-piece hard hypromellose capsule.

In some embodiments of the invention, the hard shell or soft shell of the capsule comprises gelatine.

In some embodiments of the invention, the hard shell or soft shell of the capsule comprises gellan gum.

In some embodiments of the invention, the hard-shell or soft shell of the capsule comprises hydroxypropylmethyl cellulose.

In some embodiments of the invention, the hard-shell or soft shell of the capsule comprises polyvinyl alcohol.

In some embodiments of the invention, the hard-shell or soft shell of the capsule comprises starch.

In some embodiments of the invention, the hard-shell or soft shell of the capsule comprises hypromellose.

In some embodiments of the invention, the hard-shell or soft shell of the capsule comprises gellan gum and hydroxypropylmethyl cellulose (HPMC).

In some embodiments of the invention, the hard-shell or soft shell of the capsule comprises gellan gum and hydroxypropyl cellulose (HPC).

In some embodiments of the invention, the one or more surfactants having a chemical structure that includes a polyethylene glycol (PEG) moiety is present in an amount of at least 25% by weight of the one or more SEDDS.

In the present context "moiety" is given the common understanding within organic chemistry, i.e., the polyethylene glycol moiety is part of a molecule and can be identified as such within the molecule. This may be as a tail of the surfactant, being a side group of the surfactant, being part of a chain within the surfactant, etc. In the present context, a "polyethylene glycol moiety" is specifically not free polyethylene glycol.

One of the advantages of including one or more surfactants having a chemical structure that includes a polyethylene glycol (PEG) moiety as part of self-emulsifying systems according to the invention is that in vivo uptake of cannabinoids was seen to be surprisingly increased. This was highly unexpected since a high content of PEG in the surfactant was theoretically expected to result in less cell uptake due to expected high steric hindrance of a surfactant with a PEG moiety for cell uptake. However, the exact opposite was seen, which was a great surprise to the inventors.

Specifically, it was seen that the in vivo uptake of cannabinoids in rats by including a surfactant with polyethylene glycol (PEG) moiety as part of self-emulsifying systems according to the invention was especially pronounced on a short time scale as cannabinoid plasma concentration was initially very high as function of time compared to self-emulsifying systems containing other surfactants, such as surfactants without the PEG moiety, when applied in an in vivo test set-up with rats. Also, the plasma concentration was initially very high compared to the reference, Epidiolex^{®}, which system contains a high content of sesame oil, which by the way is problematic due to adverse laxative properties.

As using herein, the term "long time scales" denotes more than 240 minutes after administration of cannabinoids in vivo in rats and "short time scales" denote less than 240 minutes after administration of cannabinoids in vivo in rats. Without being bound by theory, it is believed that "short time scales" may indicate uptake in the small intestine of the rats, whereas "long time scales" may indicate uptake in the colon of rats.

However, not only was a surprisingly initial uptake seen by including one or more surfactants having a chemical structure that include a polyethylene glycol (PEG) moiety as part of self-emulsifying systems according to the invention. Also, an overall uptake of cannabinoids was seen to be high as a function of time, including a relative high uptake on a long time scale as indicated by an elevated plasma concentration of cannabinoids as function of time. Compared to the reference, Epidiolex^{®}, the overall uptake of cannabinoids, including uptake on both short and long time scales, was on average at the same level and even higher.

Furthermore, the stability of self-emulsifying systems with surfactants having a PEG moiety was seen to stabilize the cannabinoids to a surprisingly high degree, whereby degradation in the harsh environment in the gastrointestinal tract was limited.

In some embodiments of the invention, the one or more surfactants having a chemical structure that includes a polyethylene glycol (PEG) moiety is present in an amount of at least 30% by weight of the one or more SEDDS.

In some embodiments of the invention, the one or more surfactants having a chemical structure that includes a polyethylene glycol (PEG) moiety is present in an amount of at least 35% by weight of the one or more SEDDS.

In some embodiments of the invention, the one or more surfactants having a chemical structure that includes a polyethylene glycol (PEG) moiety is present in an amount of at least 40% by weight of the one or more SEDDS.

In some embodiments of the invention, the one or more surfactants having a chemical structure that includes a polyethylene glycol (PEG) moiety is present in an amount of at least 45% by weight of the one or more SEDDS.

In some embodiments of the invention, the one or more surfactants having a chemical structure that includes a polyethylene glycol (PEG) moiety is present in an amount of at least 50% by weight of the one or more SEDDS.

In some embodiments of the invention, the one or more surfactants having a chemical structure that includes a PEG moiety is present in an amount of 20 to 80% by weight of the one or more SEDDS.

In some embodiments of the invention, the one or more surfactants having a chemical structure that includes a PEG moiety is present in an amount of 25 to 75% by weight of the one or more SEDDS.

In some embodiments of the invention, the one or more surfactants having a chemical structure that includes a PEG moiety is present in an amount of 30 to 75% by weight of the one or more SEDDS.

In some embodiments of the invention, the one or more SEDDS is stable at a pH of about 1-4.

In some embodiments of the invention, the one or more SEDDS is stable at a pH of about 1-4 and operable to gastrointestinal tract delivery of the one or more cannabinoids upon release of the one or more SEDDS in the stomach.

In some embodiments of the invention, the one or more SEDDS are delivered in the stomach.

In some embodiments of the invention, the one or more SEDDS upon hydration forms an emulsion for delivery of the one or more cannabinoids to mucosal surfaces in the gastrointestinal tract.

In some embodiments of the invention, the one or more SEDDS upon hydration forms an emulsion for delivery of the one or more cannabinoids to mucosal surfaces in the small intestines of the gastrointestinal tract.

In some embodiments of the invention, the one or more SEDDS upon hydration forms an emulsion for delivery of the one or more cannabinoids to mucosal surfaces in the colon of the gastrointestinal tract.

In some embodiments of the invention, the one or more SEDDS is liquid.

In some embodiments of the invention, the one or more SEDDS comprises one or more lipids.

Within the context of the present invention, it is to be understood that lipids comprise oils, triglycerides and lipophilic compounds that are not triglycerides.

In some embodiments of the invention, the one or more SEDDS comprises one or more oils.

In some embodiments of the invention, the one or more SEDDS comprises one or more triglycerides.

In some embodiments of the invention, the one or more SEDDS comprises one or more triglycerides of vegetable origin.

In some embodiments of the invention, the one or more SEDDS comprises one or more oils selected from the group consisting of almond oil, castor oil, coconut oil, corn oil, cottonseed oil, linseed oil, olive oil, palm oil, peanut oil, rapeseed oil, safflower oil, sesame oil, soybean oil, sunflower oil, and combinations thereof.

In some embodiments of the invention, the one or more SEDDS comprises one or more triglycerides selected from one or more C4 to C14 triglycerides.

It is noted that within the context of the invention medium chained triglycerides, medium chain triglycerides, or MCT may be used interchangeably.

In some embodiments of the invention, the one or more SEDDS comprises one or more triglycerides selected from one or more C8 to C10 triglycerides.

In some embodiments of the invention, the one or more SEDDS comprises one or more triglycerides selected from one or more medium chain triglycerides.

In some embodiments of the invention, the one or more SEDDS comprises one or more triglycerides comprising a partially hydrogenated vegetable oil.

In some embodiments of the invention, the one or more SEDDS comprises one or more triglycerides comprising a fully hydrogenated vegetable oil.

In some embodiments of the invention, the one or more SEDDS comprises one or more triglycerides comprising caprylic acid in an amount of 50 to 80% by weight.

In some embodiments of the invention, the one or more SEDDS comprises one or more triglycerides comprising capric acid in an amount of 20 to 45% by weight.

In some embodiments of the invention, the one or more SEDDS comprises one or more lipophilic compounds selected from the group consisting of glyceryl caprylate, glyceryl caprate, glyceryl monocaprylate, glyceryl monooleate, glyceryl monostearate, glyceryl monolinoleate, polyglyceryl-3 dioleate, propylene glycol dicaprylocaprate, propylene glycol dilaurate, benzyl alcohol, alpha-tocopherol, isopropyl myristate, glycerol monocaprylocaprate, and combinations thereof.

In some embodiments of the invention, the one or more SEDDS comprises glycerol monocaprylocaprate.

In some embodiments of the invention, the one or more SEDDS is solid.

Generally, the expression "solid SEDDS" is intended to mean self-emulsifying systems that are solid and/or a semi-solid at 25 Degrees Celsius, and the expression "liquid SEDDS" is intended to mean self-emulsifying systems that are liquid and/or a semi-liquid at 25 Degrees Celsius.

In some embodiments of the invention, the one or more SEDDS comprises one or more lipids in an amount of 1 to 50% by weight of the formulation.

In some embodiments of the invention, the one or more SEDDS comprises one or more lipids in an amount of 5 to 50% by weight of the one or more SEDDS.

In some embodiments of the invention, the one or more surfactants having a chemical structure that includes a polyethylene glycol (PEG) moiety is selected from the group consisting of sugar-lipid based surfactants with PEG modification, PEG containing polymer based surfactants, PEGylated emulsifiers, and combinations thereof.

In some embodiments of the invention, the one or more surfactants having a chemical structure that includes a polyethylene glycol (PEG) moiety is sugar-lipid based surfactants with PEG modification selected from the group consisting of polyoxyethylene (20) sorbitan monolaurate, polyoxyethylene (20) sorbitan monooleate, polyoxyethylene (20) sorbitan monopalmitate, polyoxyethylene (20) sorbitan monostearate, polyoxyethylene (20) sorbitan trioleate, and combinations thereof.

In some embodiments of the invention, the one or more surfactants having a chemical structure that includes a polyethylene glycol (PEG) moiety is PEG containing polymer based surfactants selected from the group consisting of poloxamer 124, poloxamer 188, poloxamer 338, poloxamer 407, and combinations thereof.

In some embodiments of the invention, the one or more surfactants having a chemical structure that includes a polyethylene glycol (PEG) moiety is PEGylated emulsifiers selected from the group consisting of d-alpha-tocopheryl polyethylene glycol succinate, d-alpha-tocopheryl PEG-1000 succinate, PEG-15 hydroxystearate, PEG-30 castor oil, PEG-32 lauroyl glycerides, PEG-32 stearoyl glycerides, PEG-35 castor oil, PEG-40 hydrogenated castor oil, PEG-6 lauroyl glycerides, PEG-6 linoleoyl glycerides, PEG-6 oleoyl glycerides, PEG-8 caprylic/capric glycerides, polyoxyl 20 cetostearyl ether, polyoxyethylene-laurylether, polyoxyethylene-23-laurylether, and combinations thereof.

In some embodiments of the invention, the one or more surfactants having a chemical structure that includes a polyethylene glycol (PEG) moiety comprises PEG-40 hydrogenated castor oil

In some embodiments of the invention, the one or more surfactants having a chemical structure that includes a polyethylene glycol (PEG) moiety comprises poloxamer 188.

In some embodiments of the invention, the one or more surfactants having a mean HLB-value of more than 6.

In some embodiments of the invention, the one or more surfactants is having a mean HLB-value of more than 9.

In some embodiments of the invention, the one or more surfactants is selected from the group consisting of PEG-35 castor oil, PEG-6 oleoyl glycerides, PEG-6 linoleoyl glycerides, PEG-8 caprylic/capric glyceride, sorbitan monolaurate, sorbitan monooleate, polyoxyethylene (20) sorbitan monolaurate, polyoxyethylene (60) sorbitan monostearate, polyoxyethylene (80) sorbitan monooleate, lauroylpoloxyl-32 glycerides, stearoyl polyoxyl-32 glycerides, polyoxyl-32 stearate, propylene glycol mono laurate, propylene glycol di laurate, and mixtures and combinations thereof.

In some embodiments of the invention, the one or more SEDDS further comprises one or more hydrophilic solvents.

In some embodiments of the invention, the one or more SEDDS further comprises one or more hydrophilic solvents in an amount of at least 5% by weight of the by weight of the one or more SEDDS.

In some embodiments of the invention, the one or more SEDDS further comprises one or more hydrophilic solvents in an amount of at least 10% by weight of the by weight of the one or more SEDDS.

In some embodiments of the invention, the one or more SEDDS further comprises one or more hydrophilic solvents in an amount of at least 20% by weight of the by weight of the one or more SEDDS.

In some embodiments of the invention, the one or more self-emulsifying systems further comprises one or more hydrophilic solvents in an amount of 10 to 50% by weight of the by weight of the one or more SEDDS.

In some embodiments of the invention, the one or more self-emulsifying systems further comprises one or more hydrophilic solvents in an amount of 10 to 40% by weight of the by weight of the one or more SEDDS.

In some embodiments of the invention, the one or more self-emulsifying systems further comprises one or more hydrophilic solvents in an amount of 10 to 30% by weight of the by weight of the one or more SEDDS.

In some embodiments of the invention, the one or more SEDDS further comprises one or more hydrophilic solvents selected from the group consisting of propylene glycol, acetyl tributyl citrate, acetyl triethyl citrate, dimethyl sulfoxide, ethanol, ethyl oleate, glycerol triacetate, diethylene glycol monoethyl ether, polyethylene glycol 200, polyethylene glycol 300, polyethylene glycol 400, polyethylene glycol 600, propan-2-ol, glycerol, triethyl citrate, and combinations thereof.

In some embodiments of the invention, the one or more SEDDS further comprises one or more hydrophilic solvents comprising propylene glycol.

In some embodiments of the invention, the one or more SEDDS further comprises at most 20% by weight of propylene glycol.

In some embodiments of the invention, the one or more SEDDS further comprises at most 15% by weight of propylene glycol.

In some embodiments of the invention, the one or more SEDDS further comprises at most 12% by weight of propylene glycol.

In some embodiments of the invention, the one or more SEDDS further comprises at most 10% by weight of propylene glycol.

In some embodiments of the invention, the one or more SEDDS does not include propylene glycol.

A relatively high amount of propylene glycol may tend to dissolve various capsules. An amount of above 20% by weight of propylene glycol, such as 40%, may tend to dissolve various capsules. Particularly, liquid SEDDS or liquid formulations according to the invention may be formulated with a relatively high amount of propylene glycol. Particularly, solid SEDDS according to the invention may be formulated with a relatively low amount of propylene glycol.

In some embodiments of the invention, the one or more surfactants further comprises one or more surfactants having a chemical structure that does not include a polyethylene glycol (PEG) moiety. These may also be denoted "non-PEG" surfactants".

In the present context "moiety" is given the common understanding within organic chemistry, i.e., the polyethylene glycol moiety is part of a molecule and can be identified as such within the molecule. This may be as a tail of the surfactant, being a side group of the surfactant, being part of a chain within the surfactant, etc. In the present context, the wording "the one or more surfactants comprises one or more surfactants having a chemical structure that does not include a polyethylene glycol (PEG) moiety" specifically implies that a "PEG" moiety is not present in the molecule.

One of the advantages of including one or more surfactants having a chemical structure that does not include a polyethylene glycol (PEG) moiety as part of self-emulsifying systems according to the invention is that in vivo uptake of cannabinoids was seen to be surprisingly increased, particularly at long time scales, such as more than 240 minutes after administration in vivo in rats. This was highly unexpected since a low content of PEG in the surfactant was theoretically expected to result in pronounced degradation of the surfactant, which was expected to result in a low cell uptake due to a lower content of surfactant following degradation. In fact, it was seen that degradation was increased as expected. Despite this, the in vivo uptake was very high at long time scales as seen for high level plasma concentration of cannabinoids in rats relatively long time after intake.

Specifically, it was seen that the in vivo uptake of cannabinoids by including a surfactant without a polyethylene glycol (PEG) moiety as part of self-emulsifying systems was especially pronounced at long time scales as cannabinoid plasma concentration was kept at a very high level as a function of time compared to other surfactants, such as surfactants including a PEG moiety, when applied in an in vivo test set-up with rats. Also, the plasma concentration was at the same level as the reference, Epidiolex^{®}, in terms of uptake of cannabinoids at long time scales.

However, not only was a surprisingly high uptake seen at long time scales. Also, an initial high uptake was seen at short time scales, much higher than Epidiolex^{®}.

Additionally, an overall uptake of cannabinoids was seen to be very high as a function of time by including a surfactant without a polyethylene glycol (PEG) moiety as part of self-emulsifying systems. Compared to the reference, Epidiolex^{®}, the overall uptake of cannabinoids, including uptake at short time scales and at long time scales, was on average much higher. Also, compared to PEG containing surfactants, including a surfactant without a polyethylene glycol (PEG) moiety (ie. a "non-PEG" surfactant") as part of a self-emulsifying system the overall level of uptake was much higher, including uptake at short time scales and at long time scales. This was highly surprising.

In some embodiments of the invention, the one or more surfactants further comprises one or more surfactants having a chemical structure that does not include a polyethylene glycol (PEG) moiety, the one or more surfactants having a chemical structure that does not include a PEG moiety being present in an amount of 0.1 to 20% by weight of the one or more SEDDS.

In some embodiments of the invention, the one or more surfactants further comprises one or more surfactants having a chemical structure that does not include a polyethylene glycol (PEG) moiety, the one or more surfactants having a chemical structure that does not include a PEG moiety being present in an amount of 0.1 to 15% by weight of the one or more SEDDS.

In some embodiments of the invention, the one or more surfactants further comprises one or more surfactants having a chemical structure that does not include a polyethylene glycol (PEG) moiety, the one or more surfactants having a chemical structure that does not include a PEG moiety being present in an amount of 5 to 15% by weight of the one or more SEDDS.

In some embodiments of the invention, the one or more surfactants comprises one or more surfactants having a chemical structure that does not include a polyethylene glycol (PEG) moiety being selected from the group consisting of monoglycerides, sugar-lipid based surfactants, and combinations thereof.

In some embodiments of the invention, the one or more surfactants comprises one or more surfactants having a chemical structure that does not include a polyethylene glycol (PEG) moiety comprising polyglycerol esters of fatty acids.

In some embodiments of the invention, the one or more surfactants comprises one or more surfactants having a chemical structure that does not include a polyethylene glycol (PEG) moiety being selected from the group consisting of decaglycerol mono- and dioleate, hexaglycerol octastearate, polyglycerol esters of oleic acid, propylene glycol monocaprylate, polyglyceryl-3-palmitate, propylene glycol monolaurate, triglycerol monooleate, polyglyceryl-4 oleyl ether olivate, polyglyceryl-4 laurate/sebacate, polyglyceryl-4 caprylate/caprate, and combinations thereof.

In some embodiments of the invention, the one or more surfactants comprises one or more surfactants having a chemical structure that does not include a polyethylene glycol (PEG) moiety being selected from the group consisting of sorbitan monolaurate, sorbitan monooleate, sorbitan monopalmitate, sorbitan monostearate, sorbitan trioleate, sucrose palmitate, sucrose stearate, and combinations thereof.

In some embodiments of the invention, the one or more surfactants comprises one or more surfactants having a chemical structure that does not include a polyethylene glycol (PEG) moiety comprising polyglyceryl-4 laurate/sebacate and/or polyglyceryl-4 caprylate/caprate and/or polyglyceryl-3-palmitate.

In some embodiments of the invention, the one or more surfactants comprises one or more surfactants having a chemical structure that does not include a polyethylene glycol (PEG) moiety comprising polyglyceryl-3-palmitate.

In some embodiments of the invention, the one or more surfactants comprises one or more surfactants having a chemical structure that does not include a polyethylene glycol (PEG) moiety comprising ascorbic acid palmitate.

In some embodiments of the invention, the one or more surfactants comprises one or more surfactants having a chemical structure that does not include a polyethylene glycol (PEG) moiety comprising sodium lauryl sulfate.

In some embodiments of the invention, the one or more surfactants does not comprise one or more surfactants having a chemical structure that does not include a polyethylene glycol (PEG) moiety.

In some embodiments of the invention, the formulation further comprises one or more permeation enhancers.

In some embodiments of the invention, the one or more SEDDS further comprises one or more permeation enhancers.

In some embodiments of the invention, the formulation further comprises one or more permeation enhancers in an amount of 5-15% by weight of the one or more SEDDS.

In some embodiments of the invention, the formulation further comprises one or more permeation enhancers in an amount of 5-10% by weight of the one or more SEDDS.

In some embodiments of the invention, the formulation further comprises one or more permeation enhancers in an amount of 10-15% by weight of the one or more SEDDS.

In some embodiments of the invention, the formulation further comprises one or more permeation enhancers comprising docusate sodium.

In some embodiments of the invention, the formulation further comprises antioxidants.

In some embodiments of the invention, the formulation further comprises preservatives.

In some embodiments of the invention, the one or more isolated or synthetic cannabinoids is selected from the group consisting of cannabidiol (CBD), cannabidiolic acid (CBDA), cannabidivarin (CBDV), and combinations thereof.

In some embodiments of the invention, the one or more isolated or synthetic cannabinoids is selected from the group consisting of tetrahydrocannabinol (THC), tetrahydrocannabinolic acid (THCA), tetrahydrocannabivarin (THCV), and combinations thereof.

In some embodiments of the invention, the one or more isolated or synthetic cannabinoids comprises cannabidiol (CBD).

In some embodiments of the invention, the one or more isolated or synthetic cannabinoids is selected from the group consisting of cannabidiol (CBD), cannabidiolic acid (CBDA), tetrahydrocannabinol (THC), tetrahydrocannabinolic acid (THCA), cannabigerol (CBG), cannabichromene (CBC), cannabinol (CBN), cannabielsoin (CBE), iso-tetrahydrocannabinol (iso-THC), cannabicyclol (CBL), cannabicitran (CBT), cannabivarin (CBV), tetrahydrocannabivarin (THCV), cannabidivarin (CBDV), cannabichromevarin (CBCV), cannabigerovarin (CBGV), cannabigerol monomethyl ether (CBGM), and combinations thereof.

In some embodiments of the invention, the one or more isolated or synthetic cannabinoids is an isolated cannabinoid.

In some embodiments of the invention, the one or more isolated or synthetic cannabinoids is a synthetic cannabinoid.

In some embodiments of the invention, the one or more isolated or synthetic cannabinoids is not a cannabinoid distillate with a cannabinoid purity of more than 80%.

In some embodiments of the invention, the purity of isolated cannabinoids is more than 95%. In some embodiments of the invention, the purity of isolated cannabinoids is more than 98%. In some embodiments of the invention, the purity of isolated cannabinoids is more than 99%.

In some embodiments of the invention, the one or more isolated or synthetic cannabinoids is not a cannabinoid extract with a cannabinoid purity of less than 80%.

In some embodiments of the invention, the one or more isolated or synthetic cannabinoids is present in an amount of at least 10 mg.

In some embodiments of the invention, the one or more isolated or synthetic cannabinoids is present in an amount of at least 20 mg.

In some embodiments of the invention, the one or more isolated or synthetic cannabinoids is present in an amount of at least 30 mg.

In some embodiments of the invention, the one or more isolated or synthetic cannabinoids is present in an amount of at least 40 mg.

In some embodiments of the invention, the one or more isolated or synthetic cannabinoids is present in an amount of at least 60 mg.

In some embodiments of the invention, the one or more isolated or synthetic cannabinoids is present in an amount of at least 75 mg.

In some embodiments of the invention, the one or more isolated or synthetic cannabinoids is present in an amount of at least 100 mg.

In some embodiments of the invention, the one or more isolated or synthetic cannabinoids is present in an amount of at least 150 mg.

In some embodiments of the invention, the one or more isolated or synthetic cannabinoids is present in an amount of at least 200 mg.

In some embodiments of the invention, the one or more isolated or synthetic cannabinoids is present in an amount of at least 250 mg.

In some embodiments of the invention, the one or more isolated or synthetic cannabinoids is present in an amount of at least 300 mg.

In some embodiments of the invention, the one or more isolated or synthetic cannabinoids is present in an amount of at least 400 mg.

In some embodiments of the invention, the one or more isolated or synthetic cannabinoids is present in an amount of at least 500 mg.

In some embodiments of the invention, the one or more isolated or synthetic cannabinoids is present in an amount of at least 600 mg.

In some embodiments of the invention, the one or more isolated or synthetic cannabinoids is present in an amount of 50-100 mg.

In some embodiments of the invention, the capsule further comprises one or more solid carriers selected from the group consisting of silica, microcrystalline cellulose, cellulose, silicified microcrystalline cellulose, clay, talc, starch, pregelatinized starch, calcium carbonate, dicalcium phosphate, modified calcium carbonate, magnesium carbonate, magnesium aluminometasilicate, hyper porous silica, and mixtures thereof.

In some embodiments of the invention, the capsule further comprises one or more solid carriers comprising one or more microcrystalline cellulose carriers.

In some embodiments of the invention, the capsule further comprises one or more solid carriers in an amount of above 10% by weight of the formulation.

In some embodiments of the invention, the capsule further comprises one or more solid carriers in an amount of 10% to 70% by weight of the formulation.

In some embodiments of the invention, the capsule further comprises one or more solid carriers in an amount of 10% to 60% by weight of the formulation.

In some embodiments of the invention, the capsule further comprises one or more solid carriers in an amount of 20% to 60% by weight of the formulation.

In some embodiments of the invention, the capsule further comprises one or more solid carriers in an amount of 10% to 50% by weight of the formulation.

In some embodiments of the invention, the capsule further comprises one or more solid carriers in an amount of 10% to 40% by weight of the formulation.

In some embodiments of the invention, the one or more isolated or synthetic cannabinoids is fully contained in the one or more SEDDS.

In some embodiments of the invention, the formulation comprises one or more cannabinoids separate from the one or more SEDDS.

In some embodiments of the invention, the one or more SEDDS comprises the one or more isolated or synthetic cannabinoids and one or more lipids in a weight ratio of cannabinoid to lipid of 10:1 to 1:10.

In some embodiments of the invention, the one or more SEDDS comprises the one or more isolated or synthetic cannabinoids and one or more lipids in a weight ratio of cannabinoid to lipid of 10:1 to 1:5.

In some embodiments of the invention, the one or more SEDDS comprises the one or more isolated or synthetic cannabinoids and one or more lipids in a weight ratio of cannabinoid to lipid of 8:1 to 1:2.

In some embodiments of the invention, the one or more SEDDS comprises the one or more isolated or synthetic cannabinoids and one or more lipids in a weight ratio of cannabinoid to lipid of 4:1 to 1:2.

In some embodiments of the invention, the one or more SEDDS comprises the one or more isolated or synthetic cannabinoids and one or more lipids in a weight ratio of cannabinoid to lipid of 2:1 to 1:2.

In some embodiments of the invention, the one or more SEDDS comprises the one or more isolated or synthetic cannabinoids and one or more lipids in a weight ratio of cannabinoid to lipid of 3:1 to 1:1.

In some embodiments of the invention, the one or more SEDDS comprises at least two separate SEDDS.

In some embodiments of the invention, the one or more SEDDS comprises at least two separate types of SEDDS.

In some embodiments of the invention, a first SEDDS comprises one or more surfactants having a chemical structure that includes a polyethylene glycol (PEG) moiety, and a second SEDDS comprises one or more surfactant having a chemical structure that does not have a polyethylene glycol (PEG) moiety.

In some embodiments of the invention, the absolute bioavailability of the one or more cannabinoids is at least 3.5%.

In the present context, "bioavailability" or "absolute bioavailability" is measured as the dose corrected blood plasma level relative to intravenous administration, i.e., intravenous administration would give 100% bioavailability.

In some embodiments of the invention, the absolute bioavailability of the one or more cannabinoids is at least 4.0%.

In some embodiments of the invention, the absolute bioavailability of the one or more cannabinoids is at least 4.5%.

In some embodiments of the invention, the absolute bioavailability of the one or more cannabinoids is at least 5.0%.

In some embodiments of the invention, the capsule is for use in alleviating or treating a medical condition.

In some embodiments of the invention, the capsule is for use in increasing absolute bioavailability of the one or more cannabinoids.

In the present context, "bioavailability" or "absolute bioavailability" is measured as the dose corrected blood plasma level relative to intravenous administration, i.e., intravenous administration would give 100% bioavailability.

In some embodiments of the invention, the capsule is for use in increasing absolute bioavailability of the one or more cannabinoids.

In some embodiments of the invention, the capsule is for use in increasing absolute bioavailability of the one or more cannabinoids compared to administration without SEDDS comprising PEG surfactants.

In some embodiments of the invention, the capsule is for use in increasing absolute bioavailability of the one or more cannabinoids to at least 4.0%.

In some embodiments of the invention, the capsule is for use in increasing absolute bioavailability of the one or more cannabinoids to at least 5.0%.

### DETAILED DESCRIPTION OF THE INVENTION

The verb "to comprise" as is used in this description and in the claims and its conjugations are used in its non-limiting sense to mean that items following the word are included, but items not specifically mentioned are not excluded. In addition, reference to an element by the indefinite article "a" or "an" does not exclude the possibility that more than one of the elements are present, unless the context clearly requires that there is one and only one of the elements. The indefinite article "a" or "an" thus usually means "at least one". Additionally, the words "a" and "an" when used in the present document in connection with the word comprising or containing denote "one or more." The expression "one or more" is intended to mean one, two, three or more.

As used herein, the term "approximately" or "about" in reference to a number are generally taken to include numbers that fall within a range of 5%, 10%, 15%, or 20% in either direction (greater than or less than) of the number unless otherwise stated or otherwise evident from the context (except where such number would be less than 0% or exceed 100% of a possible value).

As used herein, the term "%" and "percent" refers to percent by weight, unless otherwise is stated.

As user herein, the term "peroral delivery" is intended to mean that cannabinoids are not intended for absorption in the oral mucosa, but intended to be absorbed in the gastrointestinal tract, i.e., in the stomach, small intestine and/or colon.

As user herein, the term "direct peroral delivery" is intended to mean that the dosage form is applied direct via oral administration for peroral delivery, i.e., not intended to be absorbed in the mount, but in the gastrointestinal tract, i.e., in the stomach, small intestine and/or colon.

A "self-emulsifying drug delivery system" or "SEDDS" is a system which will form an emulsion when presented with an alternate phase with a minimum energy requirement. In contrast, an emulsifying agent, as opposed to a self-emulsifying agent, is one requiring additional energy to form an emulsion.

Due to the poor solubility of certain active ingredients in physiological fluids, it is an unmet need to solubilize cannabinoids upon mixture with the body physiological fluids to facilitate bio-absorption. To overcome low bioavailability, various lipid-based drug delivery systems and self-emulsifying systems have been developed.

Particular challenges are considered to arise when formulating a suitable high amount of cannabinoids in the dosage form for peroral delivery. Traditionally, various ingredients, such as sugar alcohols, are used as fillers in drug delivery systems. However, these have no function when applied in a dosage form for peroral delivery, and in some cases may cause problems in the stomach, such as laxative effect. Also, less cannabinoids may be applied in the formulation if fillers are present in a relatively high amount.

Particularly with respect to SEDDS, the formulation of the present invention may provide some clear benefits, both allowing a higher load of cannabinoids and at the same time offer improved gastrointestinal absorption, bioavailability and Cmax. Other advantages are also present.

Importantly, the presence of SEDDS or at least a self-emulsifying agent was seen to act in synergy with the formulation of the present invention. The presence of SEDDS or at least a self-emulsifying agent was seen to further increase the uptake of the active ingredients through mucosal surfaces, such as the gastrointestinal tract. In this respect, the gastrointestinal tract is not considered to include the oral cavity.

The route of administration in the present context is considered to be as follows. Typically, cannabinoids may be formulated in an absolute content of about 75 mg (other absolute values may be applied). The cannabinoids are typically premixed with the SEDDS according to the invention before any additional ingredients are applied, such as carriers.

When applied in capsules, the amount of carriers is generally kept relatively low. For liquid SEDDS according to the invention, an amount of carrier may be applied, such as in a relatively high amount of carrier. In certain cases, microcrystalline cellulose may be applied in case of formulation into capsules in order to minimize the risk of dissolving such capsules. For solid SEDDS according to the invention, an amount of carrier may also be applied, such as in a relatively low amount.

When applied in a liquid dosage form or solid dosage form, the amount of carriers is also generally kept relatively low. For liquid SEDDS according to the invention, an amount of carrier may be applied, such as in a relatively high amount of carrier. In certain cases, microcrystalline cellulose may be applied in case of formulation into capsules in order to minimize the risk of dissolving such capsules. For solid SEDDS according to the invention, an amount of carrier may also be applied, such as in a relatively low amount.

According to the invention, an amount of oil or triglycerides may be added in the dosage form. As will be known by a person skilled in the art, a balanced amount of such components, such as sesame oil, is to be applied in order to ensure proper delivery of cannabinoids.

In the present context, SEDDS is a solid or liquid dosage form comprising at least a surfactant and optionally a co-surfactant, characterized primarily in that said dosage form can form oil-in-water emulsion spontaneously in the oral cavity or at ambient temperature (referring generally to body temperature, namely 37° C.). When a SEDDS enters the gastrointestinal tract, it is initially self-emulsified as emulsion droplets and rapidly dispersed. The resulting microparticulate of micrometer or nanometer level can penetrate into the mucous membrane of the gastrointestinal tract, and the absorbed oil droplets enter the blood circulation, thereby significantly improving the bioavailability of the active ingredient.

The term "cannabinoid composition" is intended to mean a volume of matter comprising one or more cannabinoids. The cannabinoid composition may contain other components than cannabinoids. The cannabinoid composition may constitute cannabinoids. The cannabinoid composition may constitute one type of cannabinoids. The cannabinoid composition may constitute two types of cannabinoids. The cannabinoid composition may constitute two or more types of cannabinoids.

According to embodiments of the invention, flavors, if present, may be selected from the group consisting of coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

Antioxidants suitable for use include butylated hydroxyanisole (BHA), butylated hydroxytoluene (BHT), betacarotenes, tocopherols, acidulants such as Vitamin C (ascorbic acid or corresponding salts (ascorbates)), propyl gallate, catechins, green tea extract other synthetic and natural types or mixtures thereof.

High intensity sweetening agents can also be used according to embodiments of the invention. Preferred high intensity sweeteners include, but are not limited to sucralose, aspartame, salts of acesulfame, alitame, neotame, saccharin and its salts, cyclamic acid and its salts, glycyrrhizin, dihydrochalcones, thaumatin, monellin, monk fruit extract, advantame, stevioside and the like, alone or in combination.

Usage level of the high-intensity sweetener will vary considerably and will depend on factors such as potency of the sweetener, rate of release, desired sweetness of the product, level and type of flavor used and cost considerations. Thus, the active level of artificial sweetener may vary from about 0.001 to about 8% by weight (preferably from about 0.02 to about 8% by weight). When carriers used for encapsulation are included, the usage level of the encapsulated high-intensity sweetener will be proportionately higher.

According to preferred embodiments of the invention, the dosage form is free of high-intensity sweetener.

The invention, if desired, may include one or more fillers/texturizers including as examples, magnesium- and calcium carbonate, sodium sulphate, ground limestone, silicate compounds such as magnesium- and aluminum silicate, kaolin and clay, aluminum oxide, silicium oxide, talc, titanium oxide, mono-, di- and tri-calcium phosphates, cellulose polymers, such as wood, and combinations thereof. According to an embodiment of the invention, one preferred filler/texturizer is calcium carbonate.

According to the invention, the one or more cannabinoids may be selected from various cannabinoids.

"Cannabinoids" are a group of compounds including the endocannabinoids, the phytocannabinoids and those which are neither endocannabinoids or phytocannabinoids, hereinafter "syntho-cannabinoids".

"Endocannabinoids" are endogenous cannabinoids, which may have high affinity ligands of CB1 and CB2 receptors.

"Phytocannabinoids" are cannabinoids that originate in nature and can be found in the cannabis plant. The phytocannabinoids can be present in an extract including a botanical drug substance, isolated, or reproduced synthetically.

"Syntho-cannabinoids" are those compounds capable of interacting with the cannabinoid receptors (CB1 and/or CB2) but are not found endogenously or in the cannabis plant. Examples include WIN 55212 and rimonabant.

An "isolated phytocannabinoid" or "isolated cannabinoid" is one which has been extracted from the cannabis plant and purified to such an extent that the additional components such as secondary and minor cannabinoids and the non-cannabinoid fraction have been substantially removed.

A "synthetic cannabinoid" is one which has been produced by chemical synthesis. This term includes modifying an isolated phytocannabinoid, by, for example, forming a pharmaceutically acceptable salt thereof.

A "substantially pure" cannabinoid is defined as a cannabinoid which is present at greater than 95% (w/w) pure. More preferably greater than 96% (w/w) through 97% (w/w) thorough 98% (w/w) to 99% % (w/w) and greater.

In some embodiments, a purity of above 80% (w/w) may be applied.

A "highly purified" cannabinoid is defined as a cannabinoid that has been extracted from the cannabis plant and purified to the extent that other cannabinoids and non-cannabinoid components that are co-extracted with the cannabinoids have been substantially removed, such that the highly purified cannabinoid is greater than or equal to 95% (w/w) pure.

"Plant material" is defined as a plant or plant part (e.g. bark, wood, leaves, stems, roots, flowers, fruits, seeds, berries or parts thereof) as well as exudates, and includes material falling within the definition of "botanical raw material" in the Guidance for Industry Botanical Drug Products Draft Guidance, August 2000, US Department of Health and Human Services, Food and Drug Administration Center for Drug Evaluation and Research.

In the context of this application the terms "cannabinoid extract" or "extract of cannabinoids", which are used interchangeably, encompass "Botanical Drug Substances" derived from cannabis plant material. A Botanical Drug Substance is defined in the Guidance for Industry Botanical Drug Products Draft Guidance, August 2000, US Department of Health and Human Services, Food and Drug Administration Centre for Drug Evaluation and Research as: "A drug substance derived from one or more plants, algae, or macroscopic fungi. It is prepared from botanical raw materials by one or more of the following processes: pulverisation, decoction, expression, aqueous extraction, ethanolic extraction, or other similar processes." A botanical drug substance does not include a highly purified or chemically modified substance derived from natural sources. Thus, in the case of cannabis, "botanical drug substances" derived from cannabis plants do not include highly purified, Pharmacopoeial grade cannabinoids.

The term "Cannabis plant(s)" encompasses wild type Cannabis sativa and also variants thereof, including cannabis chemovars which naturally contain different amounts of the individual cannabinoids, Cannabis sativa subspecies indica including the variants var. indica and var. kafiristanica, Cannabis indica, Cannabis ruderalis and also plants which are the result of genetic crosses, self-crosses or hybrids thereof. The term "Cannabis plant material" is to be interpreted accordingly as encompassing plant material derived from one or more cannabis plants. For the avoidance of doubt it is hereby stated that "cannabis plant material" includes dried cannabis biomass.

Preferably the one or more cannabinoids are selected from: cannabichromene (CBC), cannabichromenic acid (CBCV), cannabidiol (CBD), cannabidiolic acid (CBDA), cannabidivarin (CBDV), cannabigerol (CBG), cannabigerol propyl variant (CBGV), cannabicyclol (CBL), cannabinol (CBN), cannabinol propyl variant (CBNV), cannabitriol (CBO), tetrahydrocannabinol (THC), tetrahydrocannabinolic acid (THCA), tetrahydrocannabivarin (THCV) and tetrahydrocannabivarinic acid (THCV A). More preferably the one or more cannabinoid is CBD or THC. This list is not exhaustive and merely details the cannabinoids which are identified in the present application for reference.

So far, more than 120 different phytocannabinoids have been identified which are within the scope of the present invention.

Cannabinoids can be split into different groups as follows: Phytocannabinoids; Endocannabinoids; and Synthetic cannabinoids.

Cannabinoid receptors can be activated by three major groups of agonist ligands, for the purposes of the present invention and whether or not explicitly denominated as such herein, lipophilic in nature and classed respectively as: endocannabinoids (produced endogenously by mammalian cells); phytocannabinoids (such as cannabidiol, produced by the cannabis plant); and, synthetic cannabinoids (such as HU-210).

Phytocannabinoids can be found as either the neutral carboxylic acid form or the decarboxylated form depending on the method used to extract the cannabinoids. For example, it is known that heating the carboxylic acid form will cause most of the carboxylic acid form to decarboxylate.

Phytocannabinoids can also occur as either the pentyl (5 carbon atoms) or propyl (3 carbon atoms) variant. For example, the phytocannabinoid THC is known to be a CB1 receptor agonist whereas the propyl variant THCV has been discovered to be a CB1 receptor antagonist meaning that it has almost opposite effects.

According to the invention, examples of phytocannabinoids may be cannabichromene (CBC), cannabichromenic acid (CBCV), cannabidiol (CBD), cannabidiolic acid (CBDA), cannabidivarin (CBDV), cannabigerol (CBG), cannabigerol propyl variant (CBGV), cannabicyclol (CBL), cannabinol (CBN), cannabinol propyl variant (CBNV), cannabitriol (CBO), tetrahydrocannabinol (THC), tetrahydrocannabinolic acid (THCA), tetrahydrocannabivarin (THCV) and tetrahydrocannabivarinic acid (THCV A). More preferably the one or more cannabinoid is CBD or THC.

The formulation according to the present invention may also comprise at least one cannabinoid selected from those disclosed in A. Douglas Kinghorn et al., Phytocannabinoids, Vol. 103, Chapter 1, pages 1-30.

Examples of endocannabinoids are molecules that activate the cannabinoid receptors within the body. Examples include 2-arachidonyl glycerol (2AG), 2-arachidonyl glyceryl ether (2AGE), arachidonyl dopamine, and arachidonyl ethanolamide (anandamide). Structurally related endogenous molecules have been identified that share similar structural features, but that display weak or no activity towards the cannabinoid receptors but are also termed endocannabinoids. Examples of these endocannabinoid lipids include 2-acyl glycerols, alkyl or alkenyl glyceryl ethers, acyl dopamines and N-acylethanolamides that contain alternative fatty acid or alcohol moieties, as well as other fatty acid amides containing different head groups. These include N-acylserines as well as many other N-acylated amino acids.

Examples of cannabinoid receptor agonists are neuromodulatory and affect short-term memory, appetite, stress response, anxiety, immune function and analgesia.

In one embodiment the cannabinoid is palmitoylethanolamide (PEA) which is an endogenous fatty acid amide belonging to the class of nuclear factor agonists.

Synthetic cannabinoids encompass a variety of distinct chemical classes: the cannabinoids structurally related to THC, the cannabinoids not related to THC, such as (cannabimimetics) including the aminoalkylindoles, 1,5-diarylpyrazoles, quinolines, and arylsulfonamides, and eicosanoids related to the endocannabinoids. All or any of these cannabinoids can be used in the present invention.

It is preferred that the formulation comprises one or two primary cannabinoids, which are preferably selected from the group consisting of, cannabidiol (CBD) or cannabidivarin (CBDV), tetrahydrocannabinol (THC), tetrahydrocannabivarin (THCV), tetrahydrocannabinolic acid (THCA), cannabigerol (CBG) and cannabidiolic acid (CBDA) or a combination thereof. It is preferred that the formulation comprises cannabidiol and/or tetrahydrocannabinol.

Preferably, the dosage form of the present invention may be used for the treatment or alleviation of pain, epilepsy, cancer, nausea, inflammation, congenital disorders, neurological disorders, oral infections, dental pain, sleep apnea, psychiatric disorders, gastrointestinal disorders, inflammatory bowel disease, appetite loss, diabetes and fibromyalgia.

In a further aspect of the present invention, the oral cannabinoid formulation is suitable for use in the treatment of conditions requiring the administration of a neuroprotectant or anti-convulsive medication.

The oral cannabinoid formulation may be for use in the treatment of seizures.

The oral cannabinoid formulation may be for use in the treatment of Dravet syndrome, Lennox Gastaut syndrome, myoclonic seizures, juvenile myoclonic epilepsy, refractory epilepsy, schizophrenia, juvenile spasms, West syndrome, infantile spasms, refractory infantile spasms, tuberous sclerosis complex, brain tumours, neuropathic pain, cannabis use disorder, post-traumatic stress disorder, anxiety, early psychosis, Alzheimer's disease, and autism.

The following non-limiting examples illustrate different variations of the present invention. The examples are meant for indicating the inventive concept; hence the mentioned examples should not be understood as exhaustive for the present. In particular, CBD is used as an exemplary compound but may also be another cannabinoid.

The following embodiments are within the scope of the present invention:
1. A capsule for peroral delivery of cannabinoids comprising a cannabinoid formulation contained in a hard shell or soft shell of the capsule, the formulation comprising:
   one or more self-emulsifying drug delivery systems (SEDDS) comprising one or more surfactants having a chemical structure that includes a polyethylene glycol (PEG) moiety in an amount of at least 20% by weight of the one or more SEDDS; and
   one or more cannabinoids comprising one or more isolated or synthetic cannabinoids in an amount of at least 10% by weight of the formulation.
2. The capsule according to embodiment 1, wherein the one or more isolated or synthetic cannabinoids is present in an amount of at least 20% by weight of the formulation.
3. The capsule according to embodiment 1 or 2, wherein the one or more isolated or synthetic cannabinoids is present in an amount of at least 30% by weight of the formulation.
4. The capsule according to any of the preceding embodiments, wherein the one or more isolated or synthetic cannabinoids is present in an amount of 20 to 50% by weight of the formulation.
5. The capsule according to any of the preceding embodiments, wherein the one or more isolated or synthetic cannabinoids is present in an amount of 20 to 40% by weight of the formulation.
6. The capsule according to any of the preceding embodiments 1, wherein the one or more isolated or synthetic cannabinoids is present in an amount of 20 to 35% by weight of the formulation.
7. The capsule according to any of the preceding embodiments, wherein the one or more isolated or synthetic cannabinoids is present in an amount of 25 to 35% by weight of the formulation.
8. The capsule according to any of the preceding embodiments, wherein the one or more SEDDS are present in an amount of at least 40% by weight of the formulation.
9. The capsule according to any of the preceding embodiments, wherein the one or more SEDDS are present in an amount of at least 50% by weight of the formulation.
10. The capsule according to any of the preceding embodiments, wherein the one or more SEDDS are present in an amount of at least 60% by weight of the formulation.
11. The capsule according to any of the preceding embodiments, wherein the one or more SEDDS are present in an amount of 40 to 80% by weight of the formulation.
12. The capsule according to any of the preceding embodiments, wherein the one or more SEDDS are present in an amount of 50 to 75% by weight of the formulation.
13. The capsule according to any of the preceding embodiments, wherein the formulation in addition to any water-soluble agents present in the one or more SEDDS comprises less than 20% by weight of water-soluble agents by weight of the formulation.
14. The capsule according to any of the preceding embodiments, wherein the formulation in addition to any water-soluble agents present in the one or more SEDDS comprises less than 10% by weight of water-soluble agents by weight of the formulation.
15. The capsule according to any of the preceding embodiments, wherein the formulation in addition to any water-soluble agents present in the one or more SEDDS does not comprise water-soluble agents.
16. The capsule according to any of the preceding embodiments, wherein the formulation does not comprise sugar alcohol or sugars.
17. The capsule according to any of the preceding embodiments, wherein the formulation does not comprise flavor.
18. The capsule according to any of the preceding embodiments, wherein the one or more SEDDS and the one or more cannabinoids constitute at least 50% by weight of the formulation.
19. The capsule according to any of the preceding embodiments, wherein the one or more SEDDS and the one or more cannabinoids constitute at least 80% by weight of the formulation.
20. The capsule according to any of the preceding embodiments, wherein the one or more SEDDS and the one or more cannabinoids constitute 100% by weight of the formulation.
21. The capsule according to any of the preceding embodiments, wherein the hard shell or soft shell of the capsule comprises gelatine.
22. The capsule according to any of the preceding embodiments, wherein the hard shell or soft shell of the capsule comprises gellan gum.
23. The capsule according to any of the preceding embodiments, wherein the hard-shell or soft shell of the capsule comprises hydroxypropylmethyl cellulose.
24. The capsule according to any of the preceding embodiments, wherein the hard-shell or soft shell of the capsule comprises gellan gum and hydroxypropylmethyl cellulose.
25. The capsule according to any of the preceding embodiments, wherein the capsule comprises a hard shell.
26. The capsule according to any of the preceding embodiments, wherein the capsule comprises a soft shell.
27. The capsule according to any of the preceding embodiments, wherein the one or more surfactants having a chemical structure that includes a polyethylene glycol (PEG) moiety is present in an amount of at least 30% by weight of the one or more SEDDS.
28. The capsule according to any of the preceding embodiments, wherein the one or more surfactants having a chemical structure that includes a polyethylene glycol (PEG) moiety is present in an amount of at least 35% by weight of the one or more SEDDS.
29. The capsule according to any of the preceding embodiments, wherein the one or more surfactants having a chemical structure that includes a polyethylene glycol (PEG) moiety is present in an amount of at least 40% by weight of the one or more SEDDS.
30. The capsule according to any of the preceding embodiments, wherein the one or more surfactants having a chemical structure that includes a polyethylene glycol (PEG) moiety is present in an amount of at least 45% by weight of the one or more SEDDS.
31. The capsule according to any of the preceding embodiments, wherein the one or more surfactants having a chemical structure that includes a polyethylene glycol (PEG) moiety is present in an amount of at least 50% by weight of the one or more SEDDS.
32. The capsule according to any of the preceding embodiments, wherein the one or more SEDDS is stable at a pH of about 1-4.
33. The capsule according to any of the preceding embodiments, wherein the one or more SEDDS is stable at a pH of about 1-4 and operable to gastrointestinal tract delivery of the one or more cannabinoids upon release of the one or more SEDDS in the stomach.
34. The capsule according to any of the preceding embodiments, wherein the one or more SEDDS are delivered in the stomach.
35. The capsule according to any of the preceding embodiments, wherein the one or more SEDDS upon hydration forms an emulsion for delivery of the one or more cannabinoids to mucosal surfaces in the gastrointestinal tract.
36. The capsule according to any of the preceding embodiments, wherein the one or more SEDDS upon hydration forms an emulsion for delivery of the one or more cannabinoids to mucosal surfaces in the small intestines of the gastrointestinal tract.
37. The capsule according to any of the preceding embodiments, wherein the one or more SEDDS upon hydration forms an emulsion for delivery of the one or more cannabinoids to mucosal surfaces in the colon of the gastrointestinal tract.
38. The capsule according to any of the preceding embodiments, wherein the one or more SEDDS is liquid.
39. The capsule according to any of the preceding embodiments, wherein the one or more SEDDS comprises one or more lipids.
40. The capsule according to any of the preceding embodiments, wherein the one or more SEDDS comprises one or more oils.
41. The capsule according to any of the preceding embodiments, wherein the one or more SEDDS comprises one or more triglycerides.
42. The capsule according to any of the preceding embodiments, wherein the one or more SEDDS comprises one or more triglycerides of vegetable origin.
43. The capsule according to any of the preceding embodiments, wherein the one or more SEDDS comprises one or more oils selected from the group consisting of almond oil, castor oil, coconut oil, corn oil, cottonseed oil, linseed oil, olive oil, palm oil, peanut oil, rapeseed oil, safflower oil, sesame oil, soybean oil, sunflower oil, and combinations thereof.
44. The capsule according to any of the preceding embodiments, wherein the one or more SEDDS comprises one or more triglycerides selected from one or more C4 to C14 triglycerides.
45. The capsule according to any of the preceding embodiments, wherein the one or more SEDDS comprises one or more triglycerides comprising a partially hydrogenated vegetable oil.
46. The capsule according to any of the preceding embodiments, wherein the one or more SEDDS comprises one or more triglycerides comprising a fully hydrogenated vegetable oil.
47. The capsule according to any of the preceding embodiments, wherein the one or more SEDDS comprises one or more triglycerides comprising caprylic acid in an amount of 50 to 80% by weight.
48. The capsule according to any of the preceding embodiments, wherein the one or more SEDDS comprises one or more triglycerides comprising capric acid in an amount of 20 to 45% by weight.
49. The capsule according to any of the preceding embodiments, wherein the one or more SEDDS comprises one or more lipophilic compounds selected from the group consisting of glyceryl caprylate, glyceryl caprate, glyceryl monocaprylate, glyceryl monooleate, glyceryl monostearate, glyceryl monolinoleate, polyglyceryl-3 dioleate, propylene glycol dicaprylocaprate, propylene glycol dilaurate, benzyl alcohol, alpha-tocopherol, isopropyl myristate, glycerol monocaprylocaprate, and combinations thereof.
50. The capsule according to any of the preceding embodiments, wherein the one or more SEDDS comprises one or more lipids in an amount of 1 to 50% by weight of the formulation.
51. The capsule according to any of the preceding embodiments, wherein the one or more SEDDS comprises one or more lipids in an amount of 5 to 50% by weight of the one or more SEDDS.
52. The capsule according to any of the preceding embodiments, wherein the one or more surfactants having a chemical structure that includes a polyethylene glycol (PEG) moiety is selected from the group consisting of sugar-lipid based surfactants with PEG modification, PEG containing polymer based surfactants, PEGylated emulsifiers, and combinations thereof.
53. The capsule according to any of the preceding embodiments, wherein the one or more surfactants having a chemical structure that includes a polyethylene glycol (PEG) moiety is sugar-lipid based surfactants with PEG modification selected from the group consisting of polyoxyethylene (20) sorbitan monolaurate, polyoxyethylene (20) sorbitan monooleate, polyoxyethylene (20) sorbitan monopalmitate, polyoxyethylene (20) sorbitan monostearate, polyoxyethylene (20) sorbitan trioleate, and combinations thereof.
54. The capsule according to any of the preceding embodiments, wherein the one or more surfactants having a chemical structure that includes a polyethylene glycol (PEG) moiety is PEG containing polymer based surfactants selected from the group consisting of poloxamer 124, poloxamer 188, poloxamer 338, poloxamer 407, and combinations thereof.
55. The capsule according to any of the preceding embodiments, wherein the one or more surfactants having a chemical structure that includes a polyethylene glycol (PEG) moiety is PEGylated emulsifiers selected from the group consisting of d-alpha-tocopheryl polyethylene glycol succinate, d-alpha-tocopheryl PEG-1000 succinate, PEG-15 hydroxystearate, PEG-30 castor oil, PEG-32 lauroyl glycerides, PEG-32 stearoyl glycerides, PEG-35 castor oil, PEG-40 hydrogenated castor oil, PEG-6 lauroyl glycerides, PEG-6 linoleoyl glycerides, PEG-6 oleoyl glycerides, PEG-8 caprylic/capric glycerides, polyoxyl 20 cetostearyl ether, polyoxyethylene-laurylether, polyoxyethylene-23-laurylether, and combinations thereof.
56. The capsule according to any of the preceding embodiments, wherein the one or more surfactants having a chemical structure that includes a polyethylene glycol (PEG) moiety comprises poloxamer 188.
57. The capsule according to any of the preceding embodiments, wherein the one or more surfactants having a mean HLB-value of more than 6.
58. The capsule according to any of the preceding embodiments, wherein the one or more SEDDS further comprises one or more hydrophilic solvents.
59. The capsule according to any of the preceding embodiments, wherein the one or more SEDDS further comprises one or more hydrophilic solvents in an amount of at least 5% by weight of the by weight of the one or more SEDDS.
60. The capsule according to any of the preceding embodiments, wherein the one or more SEDDS further comprises one or more hydrophilic solvents in an amount of at least 10% by weight of the by weight of the one or more SEDDS.
61. The capsule according to any of the preceding embodiments, wherein the one or more SEDDS further comprises one or more hydrophilic solvents selected from the group consisting of propylene glycol, acetyl tributyl citrate, acetyl triethyl citrate, dimethyl sulfoxide, ethanol, ethyl oleate, glycerol triacetate, diethylene glycol monoethyl ether, polyethylene glycol 200, polyethylene glycol 300, polyethylene glycol 400, polyethylene glycol 600, propan-2-ol, glycerol, triethyl citrate, and combinations thereof.
62. The capsule according to any of the preceding embodiments, wherein the one or more SEDDS further comprises one or more hydrophilic solvents comprising propylene glycol.
63. The capsule according to any of the preceding embodiments, wherein the one or more SEDDS further comprises at most 20% by weight of propylene glycol.
64. The capsule according to any of the preceding embodiments, wherein the one or more SEDDS further comprises at most 15% by weight of propylene glycol.
65. The capsule according to any of the preceding embodiments, wherein the one or more SEDDS does not include propylene glycol.
66. The capsule according to any of the preceding embodiments, wherein the one or more surfactants further comprises one or more surfactants having a chemical structure that does not include a polyethylene glycol (PEG) moiety.
67. The capsule according to any of the preceding embodiments, wherein the one or more surfactants further comprises one or more surfactants having a chemical structure that does not include a polyethylene glycol (PEG) moiety, the one or more surfactants having a chemical structure that does not include a PEG moiety being present in an amount of 0.1 to 20% by weight of the one or more SEDDS.
68. The capsule according to any of the preceding embodiments, wherein the one or more surfactants further comprises one or more surfactants having a chemical structure that does not include a polyethylene glycol (PEG) moiety, the one or more surfactants having a chemical structure that does not include a PEG moiety being present in an amount of 0.1 to 15% by weight of the one or more SEDDS.
69. The capsule according to any of the preceding embodiments, wherein the one or more surfactants further comprises one or more surfactants having a chemical structure that does not include a polyethylene glycol (PEG) moiety, the one or more surfactants having a chemical structure that does not include a PEG moiety being present in an amount of 5 to 15% by weight of the one or more SEDDS.
70. The capsule according to any of the preceding embodiments, wherein the one or more surfactants comprises one or more surfactants having a chemical structure that does not include a polyethylene glycol (PEG) moiety being selected from the group consisting of monoglycerides, sugar-lipid based surfactants, and combinations thereof.
71. The capsule according to any of the preceding embodiments, wherein the one or more surfactants comprises one or more surfactants having a chemical structure that does not include a polyethylene glycol (PEG) moiety comprising polyglycerol esters of fatty acids.
72. The capsule according to any of the preceding embodiments, wherein the one or more surfactants comprises one or more surfactants having a chemical structure that does not include a polyethylene glycol (PEG) moiety being selected from the group consisting of decaglycerol mono- and dioleate, hexaglycerol octastearate, polyglycerol esters of oleic acid, propylene glycol monocaprylate, polyglyceryl-3-palmitate, propylene glycol monolaurate, triglycerol monooleate, polyglyceryl-4 oleyl ether olivate, polyglyceryl-4 laurate/sebacate, polyglyceryl-4 caprylate/caprate, and combinations thereof.
73. The capsule according to any of the preceding embodiments, wherein the one or more surfactants comprises one or more surfactants having a chemical structure that does not include a polyethylene glycol (PEG) moiety being selected from the group consisting of sorbitan monolaurate, sorbitan monooleate, sorbitan monopalmitate, sorbitan monostearate, sorbitan trioleate, sucrose palmitate, sucrose stearate, and combinations thereof.
74. The capsule according to any of the preceding embodiments, wherein the one or more surfactants comprises one or more surfactants having a chemical structure that does not include a polyethylene glycol (PEG) moiety comprising polyglyceryl-4 laurate/sebacate and/or polyglyceryl-4 caprylate/caprate and/or polyglyceryl-3-palmitate.
75. The capsule according to any of the preceding embodiments, wherein the one or more surfactants comprises one or more surfactants having a chemical structure that does not include a polyethylene glycol (PEG) moiety comprising ascorbic acid palmitate.
76. The capsule according to any of the preceding embodiments, wherein the one or more surfactants comprises one or more surfactants having a chemical structure that does not include a polyethylene glycol (PEG) moiety comprising sodium lauryl sulfate.
77. The capsule according to any of the preceding embodiments, wherein the one or more surfactants does not comprise one or more surfactants having a chemical structure that does not include a polyethylene glycol (PEG) moiety.
78. The capsule according to any of the preceding embodiments, wherein the formulation further comprises one or more permeation enhancers.
79. The capsule according to any of the preceding embodiments, wherein the formulation further comprises one or more permeation enhancers in an amount of 5-15% by weight of the one or more SEDDS.
80. The capsule according to any of the preceding embodiments, wherein the formulation further comprises one or more permeation enhancers comprising docusate sodium.
81. The capsule according to any of the preceding embodiments, wherein the one or more isolated or synthetic cannabinoids is selected from the group consisting of cannabidiol (CBD), cannabidiolic acid (CBDA), cannabidivarin (CBDV), and combinations thereof.
82. The capsule according to any of the preceding embodiments, wherein the one or more isolated or synthetic cannabinoids is selected from the group consisting of tetrahydrocannabinol (THC), tetrahydrocannabinolic acid (THCA), tetrahydrocannabivarin (THCV), and combinations thereof.
83. The capsule according to any of the preceding embodiments, wherein the one or more isolated or synthetic cannabinoids comprises cannabidiol (CBD).
84. The capsule according to any of the preceding embodiments, wherein the one or more isolated or synthetic cannabinoids is selected from the group consisting of cannabidiol (CBD), cannabidiolic acid (CBDA), tetrahydrocannabinol (THC), tetrahydrocannabinolic acid (THCA), cannabigerol (CBG), cannabichromene (CBC), cannabinol (CBN), cannabielsoin (CBE), iso-tetrahydrocannabinol (iso-THC), cannabicyclol (CBL), cannabicitran (CBT), cannabivarin (CBV), tetrahydrocannabivarin (THCV), cannabidivarin (CBDV), cannabichromevarin (CBCV), cannabigerovarin (CBGV), cannabigerol monomethyl ether (CBGM), and combinations thereof.
85. The capsule according to any of the preceding embodiments, wherein the one or more isolated or synthetic cannabinoids is an isolated cannabinoid.
86. The capsule according to any of the preceding embodiments, wherein the one or more isolated or synthetic cannabinoids is a synthetic cannabinoid.
87. The capsule according to any of the preceding embodiments, w wherein the one or more isolated or synthetic cannabinoids is not a cannabinoid distillate with a cannabinoid purity of more than 80%.
88. The capsule according to any of the preceding embodiments, wherein the one or more isolated or synthetic cannabinoids is not a cannabinoid extract with a cannabinoid purity of less than 80%.
89. The capsule according to any of the preceding embodiments, further comprising one or more solid carriers selected from the group consisting of silica, microcrystalline cellulose, cellulose, silicified microcrystalline cellulose, clay, talc, starch, pregelatinized starch, calcium carbonate, dicalcium phosphate, modified calcium carbonate, magnesium carbonate, magnesium aluminometasilicate, hyper porous silica, and mixtures thereof.
90. The capsule according to any of the preceding embodiments, further comprising one or more solid carriers comprising one or more microcrystalline cellulose carriers.
91. The capsule according to any of the preceding embodiments, further comprising one or more solid carriers in an amount of above 10% by weight of the formulation.
92. The capsule according to any of the preceding embodiments, wherein the one or more isolated or synthetic cannabinoids is fully contained in the one or more SEDDS.
93. The capsule according to any of the preceding embodiments, wherein the formulation comprises one or more cannabinoids separate from the one or more SEDDS.
94. The capsule according to any of the preceding embodiments, wherein the one or more SEDDS comprises the one or more isolated or synthetic cannabinoids and one or more lipids in a weight ratio of cannabinoid to lipid of 10:1 to 1:10.
95. The capsule according to any of the preceding embodiments, wherein the one or more SEDDS comprises the one or more isolated or synthetic cannabinoids and one or more lipids in a weight ratio of cannabinoid to lipid of 10:1 to 1:5.
96. The capsule according to any of the preceding embodiments, wherein the one or more SEDDS comprises the one or more isolated or synthetic cannabinoids and one or more lipids in a weight ratio of cannabinoid to lipid of 8:1 to 1:2.
97. The capsule according to any of the preceding embodiments, wherein the one or more SEDDS comprises the one or more isolated or synthetic cannabinoids and one or more lipids in a weight ratio of cannabinoid to lipid of 4:1 to 1:2.
98. The capsule according to any of the preceding embodiments, wherein the one or more SEDDS comprises the one or more isolated or synthetic cannabinoids and one or more lipids in a weight ratio of cannabinoid to lipid of 2:1 to 1:2.
99. The capsule according to any of the preceding embodiments, wherein the one or more SEDDS comprises the one or more isolated or synthetic cannabinoids and one or more lipids in a weight ratio of cannabinoid to lipid of 3:1 to 1:1.
100. The capsule according to any of the preceding embodiments, wherein the one or more SEDDS comprises at least two separate SEDDS.
101. The capsule according to any of the preceding embodiments, wherein the one or more SEDDS comprises at least two separate types of SEDDS.
102. The capsule according to any of the preceding embodiments, wherein a first SEDDS comprises one or more surfactants having a chemical structure that includes a polyethylene glycol (PEG) moiety, and a second SEDDS comprises one or more surfactant having a chemical structure that does not have a polyethylene glycol (PEG) moiety.
103. The capsule according to any of the preceding embodiments, wherein the absolute bioavailability of the one or more cannabinoids is at least 4.0%.
104. The capsule according to any of the preceding embodiments, wherein the absolute bioavailability of the one or more cannabinoids is at least 5.0%.
105. The capsule according to any of the preceding embodiments for use in alleviating or treating a medical condition.
106. The capsule according to any of the preceding embodiments for use in increasing absolute bioavailability of the one or more cannabinoids.
107. The capsule according to any of the preceding embodiments for use in increasing absolute bioavailability of the one or more cannabinoids to at least 4.0%.
108. The capsule according to any of the preceding embodiments for use in increasing absolute bioavailability of the one or more cannabinoids to at least 5.0%.

### EXAMPLES

### Example 1

### Liquid self-emulsifying systems with isolated CBD and PEG and non-PEG surfactants

A cannabinoid powder composition comprising CBD isolate from cannabis plant tissues (phytocannabinoid) with a 99% content of CBD provided by Medical Hemp (batch number MH B18592) in an amount of about 300 g was sieved through a 600 microns sieve. The CBD was added to about 700 g of a composition comprising lipid, surfactant, and hydrophilic solvent (SEDDS compositions A-Z). For each SEDDS composition a total of 1000 g mixture was made to produce loaded SEDDS A30-Z30 in which the CBD content was about 300 mg/g.

The above procedure was repeated with addition of CBD to SEDDS compositions A-Z to produce loaded SEDDS A25-Z25 in which the CBD content was about 250 mg/g.

The above procedure was further repeated with addition of CBD to SEDDS compositions A-Z to produce loaded SEDDS A20-Z20 in which the CBD content was about 200 mg/g.

**Table 1: Unloaded SEDDS formulations. Raw material content denoted by weight % (wt %).**

| SEDDS | A | B | C | D | E |
|---|---|---|---|---|---|
| Raw material name | Content [%] | Content [%] | Content [%] | Content [%] | Content [%] |
| Benzyl alcohol | 14 | - | - | - | - |
| Propylene glycol | 10 | - | 31 | - | 30 |
| Alpha-tocopherol | 9 | - | - | - | - |
| Polyglyceryl-4 laurate/sebacate mixed with polyglyceryl-4 caprylate/caprate and water | 67 | - | - | - | - |
| Ascorbic acid palmitate | - | - | 2 | - | 2 |
| Sodium lauryl sulfate | - | - | 6 | - | 10 |
| Soy phosphatidylcholine | - | - | 5 | - | 6 |
| Medium chain triglycerides (C8-C10) | - | - | 20 | - | 22 |
| Glycerol monocaprylocaprate | - | - | 26 | - | 30 |
| Poloxamer 188 | - | - | 10 | - | - |
| Isopropyl myristate | - | 30 | - | 30 | - |
| Orange oil | - | 20 | - | 40 | - |
| Polyethylene lauryl ether | - | 50 | - | 30 | - |
| Total | 100 | 100 | 100 | 100 | 100 |

**Table 2: Unloaded SEDDS formulations. Raw material content denoted by weight % (wt %).**

| SEDDS | F | G | H | I | J |
|---|---|---|---|---|---|
| Raw material name | Content [%] | Content [%] | Content [%] | Content [%] | Content [%] |
| Benzyl alcohol | - | 15 | - | - | - |
| Propylene glycol | - | - | - | 40 | - |
| Alpha-tocopherol | - | 9 | - | - | - |
| Polyglyceryl-4 laurate/sebacate mixed with polyglyceryl-4 caprylate/caprate and water | 61 | - | 61 | - | 61 |
| Ascorbic acid palmitate | - | - | - | 2 | - |
| Glycerol monocaprylocaprate | - | - | - | | - |
| Poloxamer 188 | - | - | - | 39 | - |
| d-alpha-tocopheryl PEG-1000 succinate | - | 11 | - | - | - |
| Polyoxyethylene-23-laurylether | - | 65 | - | - | - |
| Polyglyceryl-3 dioleate | - | - | - | 19 | - |
| Orange oil | 22 | - | 22 | - | 22 |
| Caprylic acid | 6 | - | - | - | - |
| Capric acid | - | - | 6 | - | - |
| Oleyl alcohol | - | - | - | - | 6 |
| Ethanol | 11 | - | 11 | - | 11 |
| Total | 100 | 100 | 100 | 100 | 100 |

**Table 3: Unloaded SEDDS formulations. Raw material content denoted by weight % (wt %).**

| SEDDS | K | L | M | N | O |
|---|---|---|---|---|---|
| Raw material name | Content [%] | Content [%] | Content [%] | Content [%] | Content [%] |
| Propylene glycol | - | - | 40 | - | - |
| Ascorbic acid palmitate | - | - | 2 | - | - |
| Soy phosphatidylcholine | 20 | 20 | - | 30 | - |
| Glycerol monocaprylocaprate | - | - | 19 | - | - |
| Poloxamer 188 | - | - | 39 | - | - |
| d-alpha-tocopheryl PEG-1000 succinate | 20 | 20 | - | 20 | 20 |
| Polyoxyethylene lauryl ether | - | 10 | - | - | - |
| Isopropyl myristate | 30 | 30 | - | 30 | 30 |
| Orange oil | 20 | 20 | - | 20 | 40 |
| Polyoxyethylene (80) sorbitan monooleate | 10 | - | - | - | 10 |
| Total | 100 | 100 | 100 | 100 | 100 |

**Table 4: Unloaded SEDDS formulations. Raw material content denoted by weight % (wt %).**

| SEDDS | P | Q | R | S | T |
|---|---|---|---|---|---|
| Raw material name | Content [%] | Content [%] | Content [%] | Content [%] | Content [%] |
| Benzyl alcohol | 10 | - | 21 | - | - |
| Propylene glycol | - | - | 35 | 41 | 31 |
| Alpha-tocopherol | 9 | - | - | - | - |
| Sodium dodecyl sulfate | - | - | 6 | - | 10 |
| Ascorbic acid palmitate | - | - | - | - | 2 |
| Soy phosphatidylcholine | 5 | - | 9 | - | 8 |
| Medium chain triglycerides (C8-C10) | - | - | - | 18 | - |
| Glycerol monocaprylocaprate | - | - | 29 | - | 10 |
| Poloxamer 188 | - | - | - | 41 | 20 |
| d-alpha-tocopheryl PEG-1000 succinate | 10 | 20 | - | - | - |
| Polyoxyethylene lauryl ether | 53 | 10 | - | - | - |
| Isopropyl myristate | 13 | 30 | - | - | - |
| Orange oil | - | 40 | - | - | - |
| Caprylic acid | - | - | - | - | 19 |
| Total | 100 | 100 | 100 | 100 | 100 |

**Table 5: Unloaded SEDDS formulations. Raw material content denoted by weight % (wt %).**

| SEDDS | U | V | X | Y | Z |
|---|---|---|---|---|---|
| Raw material name | Content [%] | Content [%] | Content [%] | Content [%] | Content [%] |
| Ethanol | 7 | 23 | - | - | - |
| Propylene glycol | - | - | 30 | 30 | - |
| Propylene glycol dicaprylocaprate | 10 | - | - | - | - |
| Sodium dodecyl sulfate | - | - | 10 | 6 | - |
| Polyglyceryl-4 laurate/sebacate mixed with polyglyceryl-4 caprylate/caprate and water | 30 | - | - | - | - |
| Soy phosphatidylcholine | 3 | 7 | 6 | 6 | - |
| Medium chain triglycerides (C8-C10) | - | - | 22 | 20 | - |
| Glycerol monocaprylocaprate | - | - | 30 | 26 | - |
| Poloxamer 188 | - | - | - | 10 | - |
| d-alpha-tocopheryl PEG-1000 succinate | - | - | - | - | 20 |
| Polyoxyethylene lauryl ether | - | 20 | - | - | - |
| Orange oil | 20 | 20 | - | - | 20 |
| Polyglyceryl-3 dioleate | 10 | - | - | - | - |
| Polyglyceryl-4 oleyl ether olivate | 20 | - | - | - | - |
| Isopropyl myristate | - | 30 | - | - | 30 |
| Polyoxyethylene (80) sorbitan monooleate | - | - | - | - | 30 |
| Ascorbic acid palmitate | - | - | 2 | 2 | - |
| Total | 100 | 100 | 100 | 100 | 100 |

**Table 6: Variation in the content of CBD isolate (purity 99%). Sample 1143 corresponds to the procedure above yielding SEDDS with 30% load of CBD, the other samples are adjusted to the variation in contents.**

| Loaded SEDDS Number | 1140 | 1141 | 1142 | 1143 | 1144 | 1145 | 1146 |
|---|---|---|---|---|---|---|---|
| Raw material name | Content [%] | Content [%] | Content [%] | Content [%] | Content [%] | Content [%] | Content [%] |
| CBD isolate (purity 99%) | 15.0 | 20.0 | 25.0 | 30.0 | 35.0 | 40.0 | 50.0 |
| SEDDS composition | 85.0 | 80.0 | 75.0 | 70.0 | 65.0 | 60.0 | 50.0 |
| Total | 100 | 100 | 100 | 100 | 100 | 100 | 100 |

### Example 2

### Solid self-emulsifying systems with isolated CBD and PEG surfactants

A cannabinoid powder composition comprising CBD isolate from cannabis plant tissues (phytocannabinoid) with a 99% content of CBD provided by Medical Hemp (batch number MH B18592) in an amount of about 300 g was sieved through a 600 microns sieve. The CBD was added to about 700 g of a composition comprising lipid, surfactant having a chemical structure that includes a polyethylene glycol (PEG) moiety, and hydrophilic solvent (SEDDS compositions IA-IE and MA-ME). For each SEDDS composition a total of 1000 g mixture was made to produce loaded SEDDS IA30-IE30 and MA30-ME30 in which the CBD content was about 300 mg/g.

The above procedure was repeated with addition of CBD to SEDDS compositions IA-IE and MA-ME to produce loaded SEDDS IA25-IE25 and MA25-ME25 in which the CBD content was about 250 mg/g.

The above procedure was further repeated with addition of CBD to SEDDS compositions IA-IE and MA-ME to produce loaded SEDDS IA20-IE20 and MA20-ME20 in which the CBD content was about 200 mg/g.

The above procedure was further repeated with addition of CBD to SEDDS compositions IA-IE and MA-ME to produce loaded SEDDS IA10-IE10 and MA10-ME10 in which the CBD content was about 100 mg/g.

**Table 7: Unloaded SEDDS formulations. Raw material content denoted by weight % (wt %).**

| SEDDS | IA | IB | IC | ID | IE |
|---|---|---|---|---|---|
| Raw material name | Content [%] | Content [%] | Content [%] | Content [%] | Content [%] |
| Ascorbyl palmitate | 2 | 2 | 2 | 2 | 2 |
| Propylene glycol | 11 | 11 | 11 | 11 | 11 |
| Medium chain triglycerides (C8-C10) | 20 | 20 | 20 | 20 | 20 |
| PEG-40 castor oil | 56 | 40 | - | - | - |
| Poloxamer 188 | - | - | 56 | 46 | 46 |
| Polyglycerol esters of fatty acids | 11 | 11 | 11 | 11 | 11 |
| Sodium lauryl sulfate | - | 10 | - | 10 | - |
| Docusate sodium | - | - | - | - | 10 |
| Total | 100 | 100 | 100 | 100 | 100 |

**Table 8: Unloaded SEDDS formulations. Raw material content denoted by weight % (wt %).**

| SEDDS | MA | MB | MC | MD | ME |
|---|---|---|---|---|---|
| Raw material name | Content [%] | Content [%] | Content [%] | Content [%] | Content [%] |
| Ascorbyl palmitate | 2 | 2 | 2 | 2 | 2 |
| Propylene glycol | 10 | 10 | 10 | 10 | 10 |
| Medium chain triglycerides (C8-C10) | 40 | 40 | 40 | 40 | 40 |
| PEG-40 castor oil | 48 | 38 | - | - | - |
| Poloxamer 188 | - | - | 48 | 38 | 38 |
| Sodium lauryl sulfate | - | 10 | - | 10 | - |
| Docusate sodium | | | | | 10 |
| Total | 100 | 100 | 100 | 100 | 100 |

**Table 9: Variation in the content of CBD isolate (purity 99%). Sample 1150 corresponds to the procedure above yielding SEDDS with 30% load of CBD, the other samples are adjusted to the variation in contents.**

| Loaded SEDDS Number | 1147 | 1148 | 1149 | 1150 | 1151 | 1152 | 1153 |
|---|---|---|---|---|---|---|---|
| Raw material name | Content [%] | Content [%] | Content [%] | Content [%] | Content [%] | Content [%] | Content [%] |
| CBD isolate (purity 99%) | 15.0 | 20.0 | 25.0 | 30.0 | 35.0 | 40.0 | 50.0 |
| SEDDS composition | 85.0 | 80.0 | 75.0 | 70.0 | 65.0 | 60.0 | 50.0 |
| Total | 100 | 100 | 100 | 100 | 100 | 100 | 100 |

### Example 3

### Solid self-emulsifying systems with isolated CBD, PEG surfactants and permeation enhancers

A cannabinoid powder composition comprising CBD isolate from cannabis plant tissues (phytocannabinoid) with a 99% content of CBD provided by Medical Hemp (batch number MH B18592) in an amount of about 300 g was sieved through a 600 microns sieve. The CBD was added to about 700 g of a composition comprising lipid, surfactant having a chemical structure that includes a polyethylene glycol (PEG) moiety, and hydrophilic solvent (SEDDS compositions ICA-ICI). For each SEDDS composition a total of 1000 g mixture was made to produce loaded SEDDS ICA30-ICI30 in which the CBD content was about 300 mg/g.

The above procedure was repeated with addition of CBD to SEDDS compositions ICA-ICI to produce loaded SEDDS ICA25-ICI25 in which the CBD content was about 250 mg/g.

The above procedure was further repeated with addition of CBD to SEDDS compositions ICA-ICI to produce loaded SEDDS ICA20-ICI20 in which the CBD content was about 200 mg/g.

The above procedure was further repeated with addition of CBD to SEDDS compositions ICA-ICI to produce loaded SEDDS ICA10-ICI10 in which the CBD content was about 100 mg/g.

**Table 10: Unloaded SEDDS formulations. Raw material content denoted by weight % (wt %).**

| SEDDS | ICA | ICB | ICC | ICD |
|---|---|---|---|---|
| Raw material name | Content [%] | Content [%] | Content [%] | Content [%] |
| Ascorbyl palmitate | 2 | 2 | 2 | 2 |
| Propylene glycol | 11 | 11 | 11 | 11 |
| Medium chain triglycerides (C8-C10) | 20 | 20 | 20 | 20 |
| Poloxamer 188 | 46 | 46 | 46 | 46 |
| Polyglycerol esters of fatty acids | 11 | 11 | 11 | 11 |
| Caprylic acid | 10 | - | - | - |
| Capric acid | - | 10 | - | - |
| Docusate Na | - | - | 10 | - |
| Lauryl sulfate Na | - | - | - | 10 |
| Total | 100 | 100 | 100 | 100 |

**Table 11: Unloaded SEDDS formulations. Raw material content denoted by weight % (wt %).**

| SEDDS | ICE | ICF | ICG | ICH | ICI |
|---|---|---|---|---|---|
| Raw material name | Content [%] | Content [%] | Content [%] | Content [%] | Content [%] |
| Ascorbyl palmitate | 2 | 2 | 2 | 2 | 2 |
| Propylene glycol | 11 | 11 | 11 | 11 | 11 |
| Medium chain triglycerides (C8-C10) | 20 | 20 | 20 | 20 | 20 |
| Poloxamer 188 | 46 | 46 | 46 | 46 | 46 |
| Polyglycerol esters of fatty acids | 11 | 11 | 11 | 11 | 11 |
| Octyl sulfate Na | 10 | - | - | - | - |
| Deoxycholate Na | - | 10 | - | - | - |
| Cholate Na | - | - | 10 | - | - |
| Taurocholate Na | - | - | - | 10 | - |
| Stearoyl-2-lactylate Na | - | - | - | | 10 |
| Total | 100 | 100 | 100 | 100 | 100 |

**Table 12: Variation in the content of CBD isolate (purity 99%). Sample 1157 corresponds to the procedure above yielding SEDDS with 30% load of CBD, the other samples are adjusted to the variation in contents.**

| Loaded SEDDS Number | 1154 | 1155 | 1156 | 1157 | 1158 | 1159 | 1160 |
|---|---|---|---|---|---|---|---|
| Raw material name | Content [%] | Content [%] | Content [%] | Content [%] | Content [%] | Content [%] | Content [%] |
| CBD isolate (purity 99%) | 15.0 | 20.0 | 25.0 | 30.0 | 35.0 | 40.0 | 50.0 |
| SEDDS composition | 85.0 | 80.0 | 75.0 | 70.0 | 65.0 | 60.0 | 50.0 |
| Total | 100 | 100 | 100 | 100 | 100 | 100 | 100 |

### Example 4

### SEDDS characterization

In the Table below, characteristic properties of loaded SEDDS from Example 1 (in 1141 or 1143 corresponding to a CBD load of 20% or 30%, respectively) and Example 3 (in 1156 corresponding to a CBD load of 25%) are outlined in terms of the droplet size of the SEDDS and the zeta potential of the SEDDS, i.e., preloaded SEDDS with CBD. Both properties affect the properties of the SEDDS for mucus penetration.

The droplet size was measured for preloaded SEDDS with CBD emulsified 1:100 (v/v) in deionized water and incubated over 4 hours at 37 Degree Celsius at 300 rpm. Values are averages (n=4). The values were measured after 4 hours.

The zeta potential was measured for preloaded SEDDS with CBD emulsified 1:100 (v/v) in deionized water and incubated over 4 hours at 37 Degree Celsius at 300 rpm. Values are averages (n=4). The values were measured after 4 hours.

**Table 13: Measures after 4 hours. R and S measured after 72 hours. G, P, IC, ID and IE measured after 24 hours.**

| SEDDS | Droplet size | Zeta potential |
|---|---|---|
| Samples | nm | mV |
| A20 | 199 | -33 |
| B20 | n.a. | n.a. |
| C30 | 187 | -1 |
| D20 | 230 | -2 |
| E30 | 187 | -1 |
| F20 | 80 | 0 |
| G20 | 150 | -2 |
| H20 | 78 | -3 |
| I30 | 132 | -4 |
| J20 | 200 | -10 |
| K20 | 250 | -4 |
| L20 | n.a. | n.a. |
| M30 | 182 | -1 |
| N20 | 250 | -1 |
| O20 | 210 | -5 |
| P20 | 230 | -2 |
| Q20 | n.a. | n.a. |
| R30 | 220 | n.a. |
| S30 | 102 | n.a. |
| T30 | 271 | -24 |
| U20 | n.a. | n.a. |
| V20 | n.a. | n.a. |
| X30 | 139 | -2 |
| Y30 | 136 | -41 |
| Z20 | 600 | -1 |
| IC25 | 350 | -14 |
| ID25 | 250 | -18 |
| IE25 | 200 | -20 |

### Example 4A

### Premix: Self-emulsifying systems with isolated CBD and carriers

Microcrystalline cellulose (Vivapur 105 from JRS Pharma) in an amount of about 1000 g was added to a Lödige high shear mixer. Thereafter, a cannabinoid powder composition comprising CBD isolate from cannabis plant tissues (phytocannabinoid) with a 99% content of CBD provided by Medical Hemp (batch number MH B18592) in an amount of about 500 g was sieved through a 600 microns sieve. The CBD was added to about 500 g of a SEDDS composition according to Example 1-3. After activation of the chopper (about 600 rpm) of the Lödige mixer, the SEDDS composition containing the CBD was added to the mixture. A total of 2 kg mixture powder premix was made in which the CBD content was about 250 mg/g. A permeation enhancer could optionally be added as outlined in Example 3.

**Table 13A: Variation in the content of CBD isolate (purity 99%). Sample 1303 corresponds to the procedure above, the other samples are adjusted to the variation in contents.**

| Premix Number | 1300 | 1301 | 1302 | 1303 | 1304 | 1305 | 1306 |
|---|---|---|---|---|---|---|---|
| Raw material name | Content [%] | Content [%] | Content [%] | Content [%] | Content [%] | Content [%] | Content [%] |
| Vivapur 105 | 20.0 | 30.0 | 40.0 | 50.0 | 60.0 | 70.0 | 80.0 |
| CBD isolate (purity 99%) | 40.0 | 35.0 | 30.0 | 25.0 | 20.0 | 15.0 | 10.0 |
| SEDDS composition | 40.0 | 35.0 | 30.0 | 25.0 | 20.0 | 15.0 | 10.0 |
| Total | 100 | 100 | 100 | 100 | 100 | 100 | 100 |

### Example 4B - Preparation of SEDDS filled into capsules

Formulations containing loaded SEDDS of Examples 1-3 and 4A were filled into capsules for peroral administration.

The standard capsules applied were:
I: "Hard Gelatin Capsules - Koni Snap", made from gelatin
II: "Vcaps Plus Capsules", made from hydroxypropylmethyl cellulose (HPMC)
III: "DRcaps Capsules", made from HPMC and minor contents of gellan gum
IV: Softgel Capsules, made from gelatin and minor contents of glycerol

### Example 4C - Preparation of SEDDS for direct peroral delivery

Formulations containing loaded SEDDS of Examples 1-3 and 4A were applied in glass containers and administered in pipettes for direct oral administration with a unit dose of 100 mg CBD. This was done both with liquid dosage forms and solid dosage forms.

### Example 5

### Degradability of selected SEDDS

Effect studies were made to establish the effect of degradability on selected SEDDS from Example 1. For SEDDS A, G, P the load of CBD in the SEDDS was 20% by weight of the SEDDS (as outlined in Example 1). For SEDDS C, E, I, M the load of CBD in the SEDDS was 30% by weight of the SEDDS (as outlined in Example 1).

The studies were made *in vitro* based on release of fatty acids (in mmole) from SEDDS formulations after treatment with lipase for 60 min (incubation time). The content of released fatty acids was an indication of degradability. A blank sample was prepared for valuation of the results.

**Table 14: Release of fatty acids measured in millimole as a function of time.**

| SEDDS | 0 min | 5 min | 10 min | 20 min | 30 min | 40 min | 50 min | 60 min |
|---|---|---|---|---|---|---|---|---|
| A20 | 0.00 | 0.04 | 0.07 | 0.10 | 0.15 | 0.18 | 0.27 | 0.32 |
| C30 | 0.00 | 0.05 | 0.08 | 0.10 | 0.13 | 0.14 | 0.15 | 0.16 |
| E30 | 0.00 | 0.06 | 0.09 | 0.13 | 0.15 | 0.16 | 0.17 | 0.18 |
| G20 | 0.00 | 0.00 | 0.00 | 0.00 | 0.00 | 0.00 | 0.00 | 0.00 |
| I30 | 0.00 | 0.00 | 0.00 | 0.00 | 0.01 | 0.01 | 0.01 | 0.01 |
| M30 | 0.00 | 0.04 | 0.05 | 0.06 | 0.07 | 0.08 | 0.08 | 0.09 |
| P20 | 0.00 | 0.00 | 0.00 | 0.00 | 0.00 | 0.00 | 0.00 | 0.00 |

The results revealed that certain SEDDS with a relatively low amount of PEG containing surfactants degraded relatively fast, such as sample A20, C30 and E30. On the other hand, SEDDS with a relatively high amount of PEG containing surfactants degraded to a less degree. This trend was as expected since the PEG moiety in the surfactants was expected to protect the SEDDS from degradation.

### Example 6

### Sensorial evaluation of selected SEDDS

Selected SEDDS were evaluated *in vivo* by a sensorial test panel. The test set-up was composed of 8 test persons in a test panel. All of the test persons were healthy individuals appointed on an objective basis according to specified requirements. The sensory analysis was performed according to ISO 4121-2003 in testing conditions following ISO 8589. The result is an average of the results of the 8 individuals.

**Table 15: Sensorial evaluation in accordance to ISO 4121-2003 in testing conditions following ISO 8589.**

| SEDDS | Sensorial evaluation | rating |
|---|---|---|
| IC | Soft, smooth and waxy mouth feel, slightly sweet and mostly neutral taste, start melting in the oral cavity after a few seconds | Pleasant |
| ID | Soft, smooth and waxy mouth feel, fast onset of melting in the oral cavity, overall neutral taste with minor bitter taste appearing after prolonged retention in the oral cavity (approx. 30-45 seconds) | Acceptable to pleasant |
| IE | Soft, smooth and waxy feeling, immediate onset of melting in the oral cavity, overall neutral taste with slight bitter taste appearing after a few seconds | Acceptable |

### Example 7

### Effect studies

Effect studies were made to establish the plasma pharmacokinetics (PK) profile in Sprague Dawley rats of selected SEDDS from Example 1. The load of CBD in the SEDDS was about 20% by weight of the SEDDS for A, P and G (as outlined in Example 1).

Samples with preloaded SEDDS with CBD (in deionized water) were administered to the rats by standard oral gavage techniques, i.e., by means of a tube delivering the SEDDS in the stomach of the rats.

The studies were made by orally administering 6.643 CBD mg/kg rat, equivalent of a human (70 kg) consuming 75 mg of CBD. The plasma CBD concentration (mg/mL) was measured after certain time intervals revealing the *in vivo* uptake of CBD. Values are averages (n=7). A reference Epidiolex^{®} was applied.

The area under the curve (AUC) was established for the periods 0 to 240 minutes as well as 0 to 1440 minutes, corresponding to the total test period. The values were applied to establish uptake in the rats at short time scales (0 to 240 minutes) and uptake in the rats at long time scales (240 to 1440 minutes) as well as to measure the total uptake of CBD in the rats over the complete time period.

Additionally, the maximum plasma concentration (Cmax) was determined.

**Table 16: The area under the curve (AUC) was established for the periods (indicated in minutes from time 0 to various time points) as mean values (n=7). The values are indicated in minutes multiplied by nanogram CBD per milliliter. Also, a percentage was calculated.**

| SEDDS | AUC 0-240 | AUC 0-1440 | AUC 0-240 | AUC 0-1440 - AUC 0-240 |
|---|---|---|---|---|
| No. | min*ng/mL | min*ng/mL | % of AUC 0-1440 | min*ng/mL |
| A20 | 4152 | 10337 | 40.2 | 6185 |
| P20 | 3706 | 8575 | 43.2 | 4869 |
| G20 | 4535 | 8482 | 53.5 | 3947 |
| Epidiolex^{®} | 1988 | 8428 | 23.6 | 6440 |

As can be seen from the results of the AUC, it is evident that G20 (having a high content of PEG containing surfactants) provides a much faster uptake of 53.5% within 240 minutes of the total uptake after 1440 minutes compared to A20 (having no content of PEG containing surfactants) with a value of 40.2% and a faster uptake than P20 (having a lower content of PEG containing surfactant than G20) with a value of 43.2% within 240 minutes of the oral uptake after 1440 minutes. This was highly surprising since it was theoretically expected that PEG containing surfactants would be exposed to steric hindrance in terms of cell uptake. However, the direct opposite occurred compared to SEDDS having less or no PEG containing surfactants.

Additionally, compared the Epidiolex^{®}, the results were even more pronounced in that the uptake for Epidiolex^{®} within 240 minutes was 23.6% of the total oral uptake after 1440 minutes. Hence, also for SEDDS containing no surfactants containing PEG (here A20 with a value of 40.2%), the uptake at short time scales was higher than that of the reference, Epidiolex^{®}.

Since the uptake at short time scales (0 to 240 minutes) was considerably higher for SEDDS with PEG containing surfactants, one perspective is to apply these SEDDS for faster uptake of cannabinoids, such as CBD, for instance in combination with SEDDS systems providing sustained uptake.

As can also be seen from the results of the AUC, the total uptake (0 to 1440 minutes) was significantly higher for A20 than all the other SEDDS and Epidiolex^{®}, i.e., a value of 10337 min* ng/mL was seen for SEDDS A20 with surfactants that did not include PEG moieties, which was considerably higher than SEDDS having a high content of surfactants with PEG (8482 for G20 and 8575 for P20), and even compared to Epidiolex^{®}. This was highly unexpected and surprising, since the degradability studies revealed in Example 5 showed that these SEDDS (with surfactants that did not include PEG moieties) were degraded. To the contrary, despite this degradation, the uptake was very high and much higher than SEDDS that did not degrade as revealed in Example 5. Hence, the total uptake (at both short and long time scales) was unexpectedly higher for SEDDS with surfactants that did not include a PEG moiety.

Furthermore, as can be seen from the results of the AUC, the uptake at long time scales (within 240 to 1440 minutes) was also significantly higher for SEDDS having surfactants that did not include PEG moieties with a value of 6185 min*ng/mL compared to SEDDS having surfactants with PEG containing moieties with values of 4869 and 3947 min*ng/mL. This is evidence for a higher uptake of those SEDDS at long time scales compared to the other SEDSS in the test. This was also highly surprising since the degradability tests in Example 5 revealed degradation of these SEDDS. Highly unexpected, the uptake was higher at long time scales despite this fact and comparable to the reference, Epidiolex^{®}. One perspective is to apply these SEDDS for more sustained uptake of cannabinoids, such as CBD, for instance in combination with SEDDS systems providing faster uptake.

**Table 17: The maximum concentration Cmax in nanogram per milliliter indicated for selected samples and a reference Epidiolex^{®}.**

| SEDDS | Cmax |
|---|---|
| No. | ng/mL |
| A20 | 34.8 |
| P20 | 30.6 |
| G20 | 35.8 |
| Epidiolex^{®} | 25.0 |

As can be seen from the results of Cmax, the maximum concentration of A20, G20 and P20 was much higher than the reference, Epidiolex^{®}. Hence, these SEDDS performed better in terms of peak concentration than the reference, i.e., the possible maximum plasma concentration level obtainable is higher. This was highly surprising, since for SEDDS having surfactants with PEG moieties (G20 and P20), it was expected that steric hindrance due to the PEG moieties would affect the uptake negatively. However, the results evidence that this was not the case. As for SEDDS having surfactants with no PEG moieties (A20), this was also unexpected in view of the degradability studies revealed in Example 5, where it was shown that those SEDDS degraded significantly.

### Example 8

### Effect studies

Additional effect studies were made to establish the plasma pharmacokinetics (PK) profile in Sprague Dawley rats for other selected SEDDS from Example 1. The load of CBD in the SEDDS was about 30% by weight of the SEDDS.

Samples with preloaded SEDDS with CBD (in deionized water) were administered to the rats by standard oral gavage techniques, i.e., by means of a tube delivering the SEDDS in the stomach of the rats.

The studies were made by orally administering 6.643 CBD mg/kg rat, equivalent of a human (70 kg) consuming 75 mg of CBD. The plasma CBD concentration (mg/mL) was measured after certain time intervals revealing the *in vivo* uptake of CBD. Values are averages (n=7). A reference Epidiolex^{®} was applied.

The area under the curve (AUC) was established for the period 0 to 1440 minutes, corresponding to the total test period. The values were applied to measure the total uptake of CBD in the rats over the complete time period.

Additionally, the maximum plasma concentration (Cmax) as well as the absolute bioavailability (compared to intravenous injection) was determined.

**Table 18: The area under the curve (AUC) was established for the period (indicated in minutes from time 0 to 1440 minutes) as mean values (n=7). The values are indicated in minutes multiplied by nanogram CBD per milliliter.**

| SEDDS | AUC 0-1440 |
|---|---|
| No. | min*ng/mL |
| I30 | 11610 |
| M30 | 13736 |
| E30 | 10664 |
| C30 | 9060 |
| Epidiolex^{®} | 10442 |

As can be seen from the results of the AUC, it is evident that I30 and M30 (having a high content of PEG containing surfactants) provide an overall uptake that is significantly higher than Epidiolex^{®}. As can be seen from the results of the AUC, it is evident that I30 and M30 (having a high content of PEG containing surfactants) provide an overall uptake that is significantly higher than E30 and C30. This was highly surprising since it was theoretically expected that PEG containing surfactants would be exposed to steric hindrance in terms of cell uptake. However, the direct opposite occurred compared to Epidiolex^{®} having no PEG containing surfactants. It could be deducted that the SEDDS having a high content of PEG containing surfactants had at least 10% improvement in bioavailability compared to Epidiolex^{®}.

**Table 19: The maximum concentration Cmax in nanogram per milliliter indicated for selected samples and a reference Epidiolex^{®}.**

| SEDDS | Cmax |
|---|---|
| No. | ng/mL |
| I30 | 44.6 |
| M30 | 64.5 |
| Epidiolex^{®} | 42.0 |

As can be seen, the SEDDS having a high content of PEG containing surfactants according to the invention were superior compared to the reference Epidiolex^{®}.

## Claims

1. A capsule for peroral delivery of cannabinoids comprising a cannabinoid formulation contained in a hard shell or soft shell of the capsule, the formulation comprising:
one or more self-emulsifying drug delivery systems (SEDDS) comprising one or more surfactants having a chemical structure that includes a polyethylene glycol (PEG) moiety in an amount of at least 20% by weight of the one or more SEDDS; and
one or more cannabinoids comprising one or more isolated or synthetic cannabinoids in an amount of at least 10% by weight of the formulation.

2. The capsule according to claim 1, wherein the one or more isolated or synthetic cannabinoids is present in an amount of 20 to 50% by weight of the formulation.

3. The capsule according to claim 1 or 2, wherein the one or more SEDDS are present in an amount of 40 to 80% by weight of the formulation.

4. The capsule according to any of the preceding claims, wherein the one or more SEDDS and the one or more cannabinoids constitute at least 80% by weight of the formulation.

5. The capsule according to any of the preceding claims, wherein the hard shell or soft shell of the capsule comprises gelatine, or gellan gum, or hydroxypropylmethyl cellulose.

6. The capsule according to any of the preceding claims, wherein the one or more surfactants having a chemical structure that includes a polyethylene glycol (PEG) moiety is present in an amount of at least 40% by weight of the one or more SEDDS.

7. The capsule according to any of the preceding claims, wherein the one or more SEDDS comprises one or more lipids and/or one or more oils.

8. The capsule according to any of the preceding claims, wherein the one or more SEDDS comprises one or more triglycerides.

9. The capsule according to any of the preceding claims, wherein the one or more SEDDS comprises one or more lipids in an amount of 5 to 50% by weight of the one or more SEDDS.

10. The capsule according to any of the preceding claims, wherein the one or more surfactants having a chemical structure that includes a polyethylene glycol (PEG) moiety is sugar-lipid based surfactants with PEG modification selected from the group consisting of polyoxyethylene (20) sorbitan monolaurate, polyoxyethylene (20) sorbitan monooleate, polyoxyethylene (20) sorbitan monopalmitate, polyoxyethylene (20) sorbitan monostearate, polyoxyethylene (20) sorbitan trioleate, and combinations thereof.

11. The capsule according to any of the preceding claims, wherein the one or more surfactants having a chemical structure that includes a polyethylene glycol (PEG) moiety is PEG containing polymer based surfactants selected from the group consisting of poloxamer 124, poloxamer 188, poloxamer 338, poloxamer 407, and combinations thereof.

12. The capsule according to any of the preceding claims, wherein the one or more surfactants having a chemical structure that includes a polyethylene glycol (PEG) moiety is PEGylated emulsifiers selected from the group consisting of d-alpha-tocopheryl polyethylene glycol succinate, d-alpha-tocopheryl PEG-1000 succinate, PEG-15 hydroxystearate, PEG-30 castor oil, PEG-32 lauroyl glycerides, PEG-32 stearoyl glycerides, PEG-35 castor oil, PEG-40 hydrogenated castor oil, PEG-6 lauroyl glycerides, PEG-6 linoleoyl glycerides, PEG-6 oleoyl glycerides, PEG-8 caprylic/capric glycerides, polyoxyl 20 cetostearyl ether, polyoxyethylene-laurylether, polyoxyethylene-23-laurylether, and combinations thereof.

13. The capsule according to any of the preceding claims, wherein the one or more surfactants having a mean HLB-value of more than 6.

14. The capsule according to any of the preceding claims, wherein the one or more SEDDS further comprises one or more hydrophilic solvents.

15. The capsule according to any of the preceding claims, wherein the one or more surfactants further comprises one or more surfactants having a chemical structure that does not include a polyethylene glycol (PEG) moiety, the one or more surfactants having a chemical structure that does not include a PEG moiety being present in an amount of 0.1 to 20% by weight of the one or more SEDDS.

16. The capsule according to any of the preceding claims, wherein the formulation further comprises one or more permeation enhancers.

17. The capsule according to any of the preceding claims, wherein the one or more isolated or synthetic cannabinoids is selected from the group consisting of cannabidiol (CBD), cannabidiolic acid (CBDA), cannabidivarin (CBDV), and combinations thereof.

18. The capsule according to any of the preceding claims, wherein the one or more isolated or synthetic cannabinoids is an isolated cannabinoid.

19. The capsule according to any of the preceding claims for use in alleviating or treating a medical condition.
